# EUROPEAN PATENT APPLICATION

(11) **EP 2 433 627 A1**
(43) Date of publication of application: **28.03.2012**
(21) Application number: 11010095.5
(22) Date of filing: 24.02.2006
(51) Int. Cl.: A61K 31/165, A61P 35/00, A61K 31/00, C08F 20/00

(54) **Facially amphiphilic polymers and oligomers, compositions thereof, and use thereof in methods of treating cancer**

(30) Priority: 25.02.2005 US 65609205 P
(62) Divisional of application: 06735949.7
(71) Applicant: The Trustees Of The University Of Pennsylvania, Philadelphia PA 19104-6283 (US); University of Massachusetts, Boston, MA 02110 (US)
(72) Inventor: Degrado, William, Moylan Pennsylvania 19065 (US); Tew, Gregory N., Amherst Massachusetts 01003 (US); Arnt, Lachelle, Pleasanton California 94588 (US)
(74) Representative: Mai, Dörr, Besier

(57) **Abstract**

The present invention discloses compositions of facially amphiphilic polymers and oligomers and their use in methods for treating or reducing cancers in humans or animals.

## Description

### BACKGROUND OF THE INVENTION

### Field of the Invention

The present invention relates to compositions of facially amphiphilic polymers and oligomers and their use in methods for treating cancers in humans or animals.

### Related Art

Antimicrobial peptides represent a large and growing class of biologically interesting compounds. They represent the first line of defense against microbes for many species including plants, insects, worms and mammals. In mammals, the peptides are produced and secreted in skin, musosal surfaces and neutrophils. There are many different classes of natural host defense peptides but, in general, most contain between 20-40 amino acid residues and adopt a facially amphiphilic secondary structure with positively charged groups segregated to one side of the secondary structure and hydrophobic groups on the opposite surface. These structures can be described as facially amphiphilic regardless of whether the secondary structure is a helix or sheet type fold. In fact, it is the overall physiochemical properties that are responsible for biological activity of these peptides and not the precise amino acid sequence.

The specificity of the cytotoxic activity of the cationic and amphiphilic peptides for bacteria over mammalian cells is most likely related to fundamental differences between the two membrane types: bacteria have a large proportion of negatively charges phospholipids headgroups on their surface, while the outer leaflet of animal cells is composed mainly of neutral lipids. Also, the presence of cholesterol in the animal cell membrane appears to reduce the activity of the antimicrobial peptides. Several mechanisms have been proposed for the process of cell killing. In the carpet mechanism, peptides aggregate parallel to the membrane surface, leading to thinning and ultimately rupture of the membrane. The so-called barrel-stave mechanism suggests that the bound peptides on the cell surface self-associate into transmembrane helical bundles that form stable aqueous pores in the membrane. A third explanation is that the peptides initially bind only to the outer leaflet of the bilayer that leads to an increase in the lateral surface pressure of the outer leaflet relative to the inner leaflet of the bilayer. This pressure imbalance results in translocation of the peptides into the interior of the bilayer with concomitant formation of transient openings in the membrane. Formation of these transient pores would allow hydration of the polar sidechains of the peptide and leakage of cellular contents. Most antimicrobial peptides probably act by more than one of these mechanisms.

It has been found that several of the antimicrobial peptides, including the magainins and human cathelicidin LL-37, are more toxic to tumor cells than normal cells. See Baker, M.A., et al., Cancer Res. 53: 3052-3057 (1993); Cruciani, R. A., et al., Proc. Natl. Acad. Sci. USA 88: 3792-3796 (1991); and Okumura, K., et al., Cancer Lett. 212: 185-194 (2004).

This preferential cytotoxic activity has been attributed to a slightly higher content of negatively charged phosphatidyl serine in the tumor cell membrane resulting in tumor cells having a slightly higher negative charge on their surface in comparison to normal animal cells. Tumor cells have other differences that may also be involved in the selectivity of the cationic amphiphilic peptides, including a higher content of O-glycosylated mucines in their cell membranes and a higher intracellular negative potential (Papo, N. and Shai, Y., Biochemistry 42: 9346-9354 (2003)).

Several synthetic peptides and peptoids have been synthesized to mimic the activity of the natural host defense proteins (DeGrado, W. F., Adv. Protein Chem., 51-124 (1988); Hamuro, Y., et al., J. Am. Chem. Soc. 121:12200-12201 (1999); Porter, E. A., et al., Nature (London) 404:565 (2000); Porter, E. A., et al., J. Am. Chem. Soc. 124:7324-7330 (2002); Liu, D. & DeGrado, W. F., J. Am. Chem. Soc. 123:7553-7559 (2001); Patch, J. A. & Barron, A. E., J. Am. Chem. Soc. 125:12092-12093 (2003); and Seurynck, S. L., et al., Biophysical Journal 84:298A-298A (2003)) and several of these these have been shown to selectively kill tumorigenic cells (Papo, N. and Shai, Y., Biochemistry 42: 9346-9354 (2003); Papo, N., et al., Cancer Res. 64:5779-5786 (2004); and Shin, S. Y., et al., Biochim. Biophys. Acta 1463:209-218 (2000)).

A series of nonpeptidic mimics of the natural antimicrobial peptides have been developed that are polymers, oligomers and small molecules comprised of non-natural building blocks. See Tew, G. N., et al., Proc. Natl. Acad. Sci. U.S.A. 99:5110-511414-16 (2002); Arnt, L., et al., J. Polym. Sci. Part A 42:3860-3864 (2004); and Liu, D., et al., Angew Chem Int Ed Engl. 43:1158-1162 (2004). See also WIPO Publ. No. WO 2004/082634; WIPO Publ. No. 02/100295, and WIPO Publ. No. 02/072007. Many of these compounds are significantly smaller and easier to prepare than the natural antimicrobial peptides and peptidic mimetics. Indeed, the shortest of these oligomers have molecular weights typical of small molecule drugs. They have the same mechanism of action as magainin, are highly potent and have a broad spectrum of activity, killing gram-positive, gram-negative and antibioticresistant human pathogens. Relative to the antimicrobial peptides, the non-peptidic mimetics are significantly less toxic towards human erythrocytes, much less expensive to prepare, and more stable. Furthermore, recent results in an animal model of bacterial infection have demonstrated robust *in vivo* efficacy for an initial set of compounds, demonstrating the ability of the compounds to access an infected tissue when administered in the bloodstream.

### BRIEF SUMMARY OF THE INVENTION

The present invention provides compositions of facially amphiphilic polymers and oligomers and methods for their use in treating cancers in humans or animals.

**R¹-[-x-A₁-y-x-A₂-y-]ₘ-R²** (I)

**R¹-[-x-A₁-x-y-A₂-y-]ₘ-R²** (II)

**R¹-[-x-A₁-x-z-y-A₂-y-z]ₘ-R²** (IV)

**R¹-[-A₁-s-A₂-s-]ₘ-R²** (V)

The facially amphiphilic polymers and oligomers of the present invention include polyamide and polyester compounds of Formulae I and II wherein x is O, NR³ or S, y is C=O, C=S or SO₂, and A₁ and A₂ are aromatic, heteroaromatic or aliphatic moieties appropriately substituted with one or more polar and/or nonpolar groups; polyurea, polycarbamate, and polycarbonate compounds of Formula IV wherein x and y are O, NR³ or S, z is C=O, C=S or SO₂, and A₁ and A₂ are aromatic, heteroaromatic or aliphatic moieties appropriately substituted with one or more polar and/or nonpolar groups; and polyaryl and polyarylalkynyl compounds of Formula V wherein s is -CH₂-, -CH₂-CH₂-, -CH=CH- , or -C≡C-, and A₁ and A₂ are aromatic or heteroaromatic moieties appropriately substituted with one or more polar and/or nonpolar groups. R¹ and R² are end groups appropriate for the specific polymer or oligomer and are as defined below.

The present invention is directed to a method of treating cancer in an animal in need thereof, the method comprising administering to the animal an effective amount of a pharmaceutical composition comprising a polymer or oligomer of the invention, or an acceptable salt or solvate thereof, and a pharmaceutically acceptable carrier or diluent.

The present invention is also directed to a method of killing or inhibiting the growth of a cancer cell, the method comprising contacting the cancer cell with an effective amount of a polymer or oligomer of the invention, or an acceptable salt or solvate thereof.

The present invention is further directed to a method of reducing cancer in an animal, the method comprising administering to the animal an effective amount of a polymer or oligomer of the invention, or an acceptable salt or solvate thereof.

The present invention is also directed to a method of inhibiting tumor growth, the method comprising contacting the tumor with an effective amount of a polymer or oligomer of the invention, or a acceptable salt or solvate thereof

The present invention is also directed to a method of treating or preventing the spread or metastasis of cancer in an animal, the method comprising administering to the animal an effective amount of a polymer or oligomer of the invention, or an acceptable salt or solvate thereof.

The present invention is further directed to a method of treating an animal afflicted with a tumor or cancer, the method comprising administering to the animal an effective amount of a polymer or oligomer of the invention, or an acceptable salt or solvate thereof.

### DETAILED DESCRIPTION OF THE INVENTION

The present invention provides non-peptidic, facially amphiphilic polymers and oligomers, pharmaceutical compositions of the facially amphiphilic polymers and oligomers, and methods of using the polymers and oligomers to treat or reduce cancer.

The polymers and oligomers of the present invention are compounds of Formulae I, II, IV, and V:

**R¹-[-x-A₁-y-x-A₂-y-]ₘ-R²** (I)

**R¹-[-x-A₁-x-y-A₂-y-]ₘ-R²** (II)

**R¹-[-x-A₁-x-z-y-A₂-y-z]ₘ-R²** (IV)

**R¹-[-A₁-s-A₂-s-]ₘ-R²** (V)

or acceptable salts or solvates thereof, wherein R¹, R², A₁, A₂, x, y, z, s and m are as defined below.

The polymers and oligomers of the present invention are capable of adopting amphiphilic conformations that allow for the segregation of polar and nonpolar regions of the molecule into different spatial regions.

The facially amphiphilic conformations adopted by the polymers and oligomers of the present invention form the basis for a number of applications. For example, the polymers and oligomers of Formulae I, II, IV, and V possess anti-microbial activity and are useful as anti-microbial agents. Use of the polymers and oligomers of Formulae I, II, and IV as anti-microbial agents is described by DeGrado *et al.* in WIPO Publication No. WO 2004/082634, the contents of which is fully incorporated by reference herein in its entirety. Use of the polymers and oligomers of Formula V as anti-microbial agents is described by DeGrado et al. in U.S. Patent Appl. No. 11/038,787, the contents of which is also fully incorporated by reference herein in its entirety.

The polymers and oligomers of Formulae I, II, IV, and V also possess anti-cancer or anti-tumorigenic activity and can also be used as anti-cancer and anti-tumor agents, *e.g.*, the polymers and oligomers can kill or inhibit the growth of cancer cells. Thus, the polymers and oligomers of Formulae I, II, IV, and V, in particular, the oligomers of Formulae I, II, IV, and V, can be used in a method of treating cancer in an animal. The polymers and oligomers of Formulae I, II, IV, and V can also be used in a method of reducing cancer in an animal, or in a method of treating or preventing the spread or metastasis of cancer in an animal, or in a method of treating an animal afflicted with cancer. The polymers and oligomers of Formulae I, II, IV, and V, in particular, the oligomers of Formulae I, II, IV, and V, can also be used in a method of killing or inhibiting the growth of a cancer cell, or in a method of inhibiting tumor growth.

The polymers and oligomers of the present invention were originally designed to mimic the antimicrobial activities of host defense peptides, which were potentially exciting therapeutic agents because of their broad spectrum of activity, rapid bacteriocidal activity, and very low incidence of development of bacterial resistance. However, significant pharmaceutical issues, including systemic toxicity and difficulty and expense of manufacturing, severely hampered clinical progress in the use of the host defense peptides as therapeutics.

The present invention directly addresses those pharmaceutical issues. For example, many of the oligomers of Formulae I, II, and IV are significantly smaller and easier to prepare than their naturally occurring counterparts. They have the same mechanism of action as magainin (a naturally occurring host defense peptide) and are approximately equipotent and as broad in their spectrum of action as magainin. However, the non-peptidic polymers and oligomers of the present invention are significantly less toxic towards human erythrocytes, much less expensive to prepare, and are expected to be much more stable *in vivo.*

The present invention discloses facially amphiphilic polymers and oligomers. Polymers are generally defined as synthetic compounds assembled from monomer subunits that are polydisperse in molecular weight, and are most commonly prepared by one-pot synthetic procedures. The term "polymer" as used herein refers to a macromolecule comprising a plurality of repeating units or monomers. The term includes homopolymers, which are formed from a single type of monomer, and copolymers, which are formed from two or more different monomers. In copolymers, the monomers may be distributed randomly (random copolymer), in alternating fashion (alternating copolymers), or in blocks (block copolymer). The polymers of the present invention are either homopolymers or alternating copolymers having about 2 monomer units to about 500 monomer units, with average molecular weights that range from about 300 Daltons to about 1,000,000 Daltons, or from about 400 Daltons to about 120,000 Daltons. Preferred polymers are those having about 5 to about 100 monomer units, with average molecular weights that range from about 1,000 Daltons to about 25,000 Daltons.

The term "oligomer" as used herein refers to as a homogenous polymer with a defined sequence and molecular weight. Modern methods of solid phase organic chemistry have allowed the synthesis of homodisperse, sequence-specific oligomers with molecular weights approaching 5,000 Daltons. An oligomer, in contrast to a polymer, has a defined sequence and molecular weight and is usually synthesized either by solid phase techniques or by step-wise solution chemistry and purified to homogeneity. Oligomers of the present invention are those having about 2 monomer units to about 25 monomer units, with molecular weights that range from about 300 Daltons to about 6,000 Daltons. Preferred oligomers are those having about 2 monomer units to about 10 monomer units, with molecular weights that range from about 300 Daltons to about 2,500 Daltons.

For the pharmaceutical applications described herein, oligomers are the preferred species because of their defined size and structure.

The term "polymer backbone," "oligomer backbone," or "backbone" as used herein refers to that portion of the polymer or oligomer which is a continuous chain comprising the bonds formed between monomers upon polymerization. The composition of the polymer or oligomer backbone can be described in terms of the identity of the monomers from which it is formed without regard to the composition of branches, or side chains, of the polymer or oligomer backbone.

The term "polymer side chain," "oligomer side chain," or "side chain" refers to portions of the monomer which, following polymerization, forms an extension of the polymer or oligomer backbone. In homopolymers and homooligomers, all the side chains are derived from the same monomer.

The terms "treat," "treated," or "treating" as used herein refers to both therapeutic treatment and prophylactic or preventative measures wherein the object is to prevent or slow down (lessen) an undesired physiological condition, disorder or disease, or obtain beneficial or desired clinical results. For purposes of this invention, beneficial or desired clinical results include, but are not limited to, alleviation of symptoms; diminishment of extent of condition, disorder or disease; stabilized (i.e., not worsening) state of condition, disorder or disease; delay in onset or slowing of condition, disorder or disease progression; amelioration of the condition, disorder or disease state or remission (whether partial or total), whether detectable or undetectable; or enhancement or improvement of condition, disorder or disease. Treatment includes eliciting a clinically significant response without excessive levels of side effects. Treatment also includes prolonging survival as compared to expected survival if not receiving treatment.

The term "animal" as used herein includes, but is not limited to, humans and non-human vertebrates such as wild, domestic and farm animals.

The term "amphiphilic" as used herein describes a three-dimensional structure having discrete hydrophobic and hydrophilic regions. An amphiphilic polymer requires the presence of both hydrophobic and hydrophilic elements along the polymer backbone. The presence of hydrophobic and hydrophilic groups is a necessary, but not sufficient, condition to produce an amphiphilic molecule, polymer or oligomer.

The term "facially amphiphilic" or "facial amphiphilicity" as used herein describes polymers or oligomers with polar (hydrophilic) and nonpolar (hydrophobic) side chains that adopt conformation(s) leading to segregation of polar and nonpolar side chains to opposite faces or separate regions of the structure or molecule.

The phrase "groups with chemically nonequivalent termini" refers to functional groups such as esters amides, sulfonamides and N-hydroxyoximes where reversing the orientation of the substituents, *e.g.* R¹C(=O)OR² *vs*. R¹O(O=)CR², produces unique chemical entities.

The polymers and oligomers of the present invention have been shown to possess anti-tumor or anti-cancer activity. Thus, the polymers and oligomers of the present invention can be used as anticancer or antineoplastic agents and, for example, can be used in a method of treating cancer in an animal.

Thus, the invention is directed to a method of treating cancer in an animal in need thereof, by administering to the animal an effective amount of a pharmaceutical composition comprising a polymer or oligomer of the invention.

The polymers and oligomers of the present invention include those of of Formula I:

**R¹-[-x-A₁-y-x-A₂-y-]ₘ-R²** (I)

or an acceptable salt or solvate thereof,
wherein:
x is NR⁸, -N(R⁸)N(R⁸)-, O, or S; y is C=O, C=S, O=S=O, or -C(=O)C(=O)- ; and R⁸ is hydrogen or alkyl;
A₁ and A₂ are independently optionally substituted arylene or optionally substituted heteroarylene, wherein:
   (i) A₁ and A₂ are independently optionally substituted with one or more polar (PL) group(s), one or more non-polar (NPL) group(s), or a combination of one or more polar (PL) group(s) and one or more non-polar (NPL) group(s); or
   (ii) A₁ is optionally substituted arylene or optionally substituted heteroarylene and A₂ is a C₃ to C₈ cycloalkyl or -(CH₂)_{q}-, wherein q is 1 to 7, wherein A₁ and A₂ are independently optionally substituted with one or more polar (PL) group(s), one or more non-polar (NPL) group(s), or a combination of one or more polar (PL) group(s) and one or more non-polar (NPL) group(s); or
   (iii) A₂ is optionally substituted arylene or optionally substituted heteroarylene, and A₁ is a C₃ to C₈ cycloalkyl or -(CH₂)_{q}-, wherein q is 1 to 7, wherein A₁ and A₂ are independently optionally substituted with one or more polar (PL) group(s), one or more non-polar (NPL) group(s), or a combination of one or more polar (PL) group(s) and one or more non-polar (NPL) group(s);
R¹ is
   (i) hydrogen, a polar (PL) group, or a non-polar (NPL) group, and R² is -x-A₁-y-R¹¹, wherein R¹¹ is hydrogen, a polar (PL) group, or a non-polar (NPL) group; or
   (ii) R¹ and R² are independently hydrogen, a polar (PL) group, or a non-polar (NPL) group; or
   (iii) R¹ and R² together are a single bond;
NPL is a nonpolar group independently selected from the group consisting of -B(OR⁴)₂ and -(NR^{3ʹ})_{q1NPL}-U^{NPL}-(CH₂)_{pNPL}-(NR^{3"})_{q2NPL}-R^{4'}, wherein:
   R³, R^{3'}, and R^{3"} are independently selected from the group consisting of hydrogen, alkyl, and alkoxy;
   R⁴ and R^{4'} are independently selected from the group consisting of hydrogen, alkyl, alkenyl, alkynyl, cycloalkyl, aryl, and heteroaryl, any of which is optionally substituted with one or more alkyl or halo groups;
   U^{NPL} is absent or selected from the group consisting of O, S, S(=O), S(=O)₂, NR³, -C(=O)-, -C(=O)-N=N-NR³-, -C(=O)-NR³-N=N-, -N=N-NR³-, -C(=N-N(R³)₂)-, -C(=NR³)-, -C(=O)O-, -C(=O)S-, -C(=S)-, -O-P(=O)₂O-, -R³O-, -R³S-, -S-C=N- and -C(=O)-NR³-O- , wherein groups with two chemically nonequivalent termini can adopt both possible orientations;
   the -(CH₂)_{pNPL}- alkylene chain is optionally substituted with one or more amino or hydroxy groups, or is unsaturated;
   pNPL is 0 to 8;
   q1NPL and q2NPL are independently 0,1 or 2;
PL is a polar group selected from the group consisting of halo, hydroxyethoxymethyl, methoxyethoxymethyl, polyoxyethylene, and -(NR^{5'})_{q1PL}-U^{PL}-(CH₂)_{pPL}-(NR^{5"})_{q2PL}- V, wherein:
   R⁵, R^{5'}, and R^{5"} are independently selected from the group consisting of hydrogen, alkyl, and alkoxy;
   U^{PL} is absent or selected from the group consisting of O, S, S(=O), S(=O)₂, NR⁵, -C(=O)-, -C(=O)-N=N-NR⁵-, -C(=O)-NR⁵-N=N-, -N=N-NR⁵-, -C(=N-N(R⁵)₂)-, -C(=NR⁵)-, -C(=O)O-, -C(=O)S-, -C(=S)-, -O-P(=O)₂O-, -R⁵O-, -R⁵S-, -S-C=N- and -C(=O)-NR⁵-O-, wherein groups with two chemically nonequivalent termini can adopt both possible orientations;
   V is selected from the group consisting of nitro, cyano, amino, hydroxy, alkoxy, alkylthio, alkylamino, dialkylamino, -NH(CH₂)ₚNH₂ wherein p is 1 to 4, -N(CH₂CH₂NH₂)₂, diazamino, amidino, guanidino, guanyl, semicarbazone, aryl, heterocycle and heteroaryl, any of which is optionally substituted with one or more of amino, halo, cyano, nitro, hydroxy, -NH(CH₂)ₚNH₂ wherein p is 1 to 4, N(CH₂CH₂NH₂)₂, amidino, guanidino, guanyl, aminosulfonyl, aminoalkoxy, aminoalkythio, lower acylamino, or benzyloxycarbonyl, wherein p is 1 to 4;
   the -(CH₂)_{pPL}- alkylene chain is optionally substituted with one or more amino or hydroxy groups, or is unsaturated;
   pPL is 0 to 8;
   q1PL and q2PL are independently 0, 1 or 2; and
m is 1 to about 500.

Polymers and oligomers of Formula I that are preferred for use in the disclosed method are those wherein x is NR⁸, O, or -N(R⁸)N(R⁸)-, and R⁸ is hydrogen or C₁-C₆ alkyl. Also preferred are those polymers and oligomers wherein y is C=O, C=S, or O=S=O. Especially preferred are those polymers and oligomers wherein x is NR⁸ and y is C=O. For example, oligomers of Formula I wherein x is NH and y is C=O are especially preferred. Also preferred are oligomers of Formula I wherein x is O and y is C=O, or wherein x is N(R⁸)N(R⁸)- and R⁸ is hydrogen, and y is C=O.

Preferred are those polymers and oligomers of Formula I wherein A₁ or A₂ are independently optionally substituted *o-, m-,* or *p*-phenylene. Those oligomers wherein A₁ or A₂ are optionally substituted *m*-phenylene are especially preferred.

Preferred polymers and oligomers of Formula I are also those wherein A₁ is optionally substituted arylene or optionally substituted heteroarylene and A₂ is a C₃ to C₈ cycloalkyl or -(CH₂)_{q}-, wherein q is 1 to 7, and wherein A₁ and A₂ are independently optionally substituted with one or more polar (PL) group(s), one or more non-polar (NPL) group(s), or a combination of one or more polar (PL) group(s) and one or more non-polar (NPL) group(s). Especially preferred are oligomers of Formula I wherein A₁ is substituted arylene and A₂ is -(CH₂)_{q}-, wherein q is 1 or 2, and wherein one of A₁ and A₂ is substituted with one or more polar (PL) group(s), and the other of A₁ and A₂ is substituted with one or more non-polar (NPL) group(s).

Also preferred are polymers and oligomers of Formula I wherein A₂ is optionally substituted arylene or optionally substituted heteroarylene, and A₁ is a C₃ to C₈ cycloalkyl or -(CH₂)_{q}-, wherein q is 1 to 7, and wherein A₁ and A₂ are independently optionally substituted with one or more polar (PL) group(s), one or more non-polar (NPL) group(s), or a combination of one or more polar (PL) group(s) and one or more non-polar (NPL) group(s). Especially preferred are oligomers of Formula I wherein A₂ is substituted arylene and A₁ is -(CH₂)_{q}-, wherein q is 1 or 2, and wherein one of A₁ and A₂ is substituted with one or more polar (PL) group(s), and the other of A₁ andr A₂ is substituted with one or more non-polar (NPL) group(s).

In some aspects of the invention, preferred polymers and oligomers of Formula I are those wherein (i) R¹ is hydrogen, a polar (PL) group, or a non-polar (NPL) group, and R² is -x-A₁-y-R¹¹, wherein R¹¹ is hydrogen, a polar (PL) group, or a non-polar (NPL) group. Especially preferred are oligomers of Formula I wherein R¹ is hydrogen, R² is -x-A₁-y-R¹¹, and R¹¹ is a polar (PL) group, for example, hydroxy.

In other aspects, preferred polymers and oligomers of Formula I are those wherein R¹ and R² are independently hydrogen, a polar (PL) group, or a non-polar (NPL) group. Especially preferred are oligomers of Formula I wherein R¹ is hydrogen, and R² is a polar group, for example, amino or hydroxy.

In some aspects of the invention, preferred polymers and oligomers of Formula I are those wherein NPL is -B(OR⁴)₂. Preferred values of R⁴ are hydrogen, C₁-C₁₀ alkyl, C₃-C₁₈ branched alkyl, C₂-C₁₀ alkenyl, C₂-C₁₀ alkynyl, C₃-C₈ cycloalkyl, C₆-C₁₀ aryl, and heteroaryl, any of which is optionally substituted with one or more C₁-C₆ alkyl or halo groups.

In other aspects, preferred polymers and oligomers of Formula I are those wherein NPL is -(NR^{3'})_{q1NPL}-U^{NPL}-(CH₂)_{pNPL}-(NR^{3"})_{q2NPL}-R^{4'}, and R³, R^{3′}, R^{3"}, R^{4'}, U^{NPL}, pNPL, q1NPL and q2NPL are as defined above.

Preferred values for each of R³, R^{3'}, and R^{3"} are hydrogen, C₁-C₆ alkyl, and C₁-C₆ alkoxy. Hydrogen is an especially preferred value for R³, R^{3'}, and R^{3"}.

Preferred values of R^{4'} are hydrogen, C₁-C₁₀ alkyl, C₃-C₁₈ branched alkyl, C₂-C₁₀ alkenyl, C₂-C₁₀ alkynyl, C₃-C₈ cycloalkyl, C₆-C₁₀ aryl, and heteroaryl, any of which is optionally substituted with one or more C₁-C₆ alkyl or halo groups. Values of R^{4'} that are more preferred are C₁-C₁₀ alkyl, C₃-C₁₈ branched alkyl, C₂-C₁₀ alkenyl, C₂-C₁₀ alkynyl, and C₆-C₁₀ aryl, especially phenyl. Especially preferred values of R^{4'} are C₁-C₁₀ alkyl and C₃-C₁₈ branched alkyl. Suitable C₁-C₁₀ alkyl and C₃-C₁₈ branched alkyl groups are methyl, ethyl, *n*-propyl, isopropyl, *n*-butyl, *tert*-butyl, *n*-pentyl, and isopentyl.

Preferred values of U^{NPL} are O, S, S(=O), S(=O)₂, NH, -C(=O)-, -C(=O)-N=N-NH-, -C(=O)-NH-N=N-, -N=N-NH-, -C(=N-N(R³)₂)-, -C(=NR³)-, -C(=O)O-, -C(=O)S-, -C(=S)-, -O-P(=O)₂O-, -R³O-, -R³S-, -S-C=N- and -C(=O)-NR³-O-, wherein groups with two chemically nonequivalent termini can adopt both possible orientations.

Values of U^{NPL} that are more preferred are O, S, NH, -C(=O)-, -C(=O)-N=N-NH-, -C(=O)-NH-N=N-, -N=N-NH-, -C(=N-N(R³)₂)-, -C(=NR³)-, - C(=O)O-, -R³S- and -R³O-. Especially preferred values of U^{NPL} are NH, - C(=O)-, O and S. Especially preferred polymers and oligomers of Formula I also are those wherein U^{NPL} is absent.

In some aspects of the invention, preferred polymers and oligomers of Formula I are those wherein U^{NPL} is -O-P(=O)₂O-.

Preferred values of pNPL are 0 to 6; values of pNPL of 0 to 4 are especially preferred, with values ofpNPL of 0 to 2 most preferred.

Preferred values of q1NPL and q2NPL are 0 or 1. Values of q1NPL and q2NPL of 0 or 1 are especially preferred, with a value of 0 being the most preferred for each of q1NPL and q2NPL.

In preferred polymers and oligomers of Formula I, the -(CH₂)_{pNPL}-alkylene chain in NPL is unsubstituted or substituted with one or more amino or hydroxy groups. More preferred are those oligomers of Formula I wherein the -(CH₂)_{pNPL}- alkylene chain in NPL is either unsubstituted or substituted with one or more amino groups.

An especially preferred value of NPL for polymers and oligomers of Formula I is C₁-C₆ alkyl or aryl C₁-C₆ alkyl. Examples of preferred values for NPL are *n*-propyl, isopropyl, *n*-butyl, *tert*-butyl, and benzyl.

In some aspects of the invention, preferred polymers and oligomers of Formula I are those wherein PL is -(NR^{5'})_{q1PL}-U^{PL}-(CH₂)_{pPL}-(NR^{5"})_{q2PL}-V, and R⁵, R^{5'}, R^{5"}, V, U^{PL}, pPL, q1PL and q2PL are as defined above.

Preferred values for R⁵, R^{5'}, and R^{5"} are hydrogen, C₁-C₆ alkyl, and C₁-C₆ alkoxy. Hydrogen is an especially preferred value for each of R⁵, R^{5'}, and R^{5"}.

Preferred values of U^{PL} are O, S, S(=O), S(=O)₂, NH, -C(=O)-, -C(=O)-N=N-NH-, -C(=O)-NH-N=N-, -N=N-NH-, -C(=N-N(R⁵)₂)-, -C(=NR⁵)-, -C(=O)O-, -C(=O)S-, -C(=S)-, -O-P(=O)₂O-, -R⁵O-, -R⁵S-, -S-C=N- and -C(=O)-NR⁵-O-, wherein groups with two chemically nonequivalent termini can adopt both possible orientations. Values of U^{PL} that are more preferred are O, S, NH, -C(=O)-, -C(=O)-N=N-NH-, -C(=O)-NH-N=N-, -N=N-NH-, -C(=N-N(R⁵)₂)-, -C(=NR⁵)-, -C(=O)O-, -R⁵Sand -R⁵O-. Especially preferred values of U^{PL} are O, S, NH, and -C(=O). Preferred polymers and oligomers of Formula I are also those wherein U^{PL} is absent.

In some aspects of the invention, preferred polymers and oligomers of Formula I are those wherein U^{PL} is -O-P(=O)₂O-.

Preferred values of V are nitro, cyano, amino, hydroxy, C₁-C₆ alkoxy, C₁-C₆ alkylthio, C₁-C₆ alkylamino, C₁-C₆ dialkylamino, -NH(CH₂)ₚNH₂ wherein p is 1 to 4, -N(CH₂CH₂NH₂)₂, diazamino, amidino, guanidino, guanyl, semicarbazone, C₆-C₁₀ aryl, heterocycle, and heteroaryl, preferably any of which is optionally substituted with one or more of amino, halo, cyano, nitro, hydroxy, -NH(CH₂)ₚNH₂, -N(CH₂CH₂NH₂)₂, amidino, guanidino, guanyl, aminosulfonyl, aminoalkoxy, lower acylamino, or benzyloxycarbonyl.

Suitable heteroaryl groups include indolyl, 3*H*-indolyl, 1*H*-isoindolyl, indazolyl, benzoxazolyl, pyridyl, and 2-aminopyridyl. Suitable heterocycle groups include piperidinyl, piperazinyl, imidazolidinyl, pyrrolidinyl, pyrazolidinyl, and morpholinyl.

Values of V that are more preferred are amino, C₁-C₆ alkylamino, C₁-C₆ dialkylamino, -NH(CH₂)ₚNH₂ wherein p is 1 to 4, -N(CH₂CH₂NH₂)₂, diazamino, amidino, guanidino, guanyl, and semicarbazone, preferably any of which is optionally substituted with one or more of amino, halo, cyano, nitro, hydroxy, -NH(CH₂)ₚNH₂, -N(CH₂CH₂NH₂)₂, amidino, guanidino, guanyl, aminosulfonyl, aminoalkoxy, lower acylamino, or benzyloxycarbonyl.

Especially preferred values of V are amino, C₁-C₆ alkylamino, -NH(CH₂)ₚNH₂ wherein p is 1 to 4, -N(CH₂CH₂NH₂)₂, diazamino, amidino, and guanidino, preferably any of which is optionally substituted with one or more of amino, halo, cyano, nitro, hydroxy, -NH(CH₂)ₚNH₂ wherein p is 1 to 4, -N(CH₂CH₂NH₂)₂, amidino, guanyl, guanidino, or aminoalkoxy. Values of V that are most preferred are amino and guanidino.

Preferred values of pPL are 0 to 6, with values of pPL of 2 to 5 especially preferred.

Preferred values of q1PL and q2PL are 0 or 1. Values of q1PL and q2PL of 0 or 1 are especially preferred, with a value of 0 being especially preferred for each of q1PL and q2PL.

In preferred polymers and oligomers of Formula I, the -(CH₂)_{pPL}-alkylene chain in PL is optionally substituted with one or more amino groups.

Preferred polymers of Formula I are those in which m is 1 to about 500. Especially preferred are those polymers of Formula I wherein m is 1 to about 100, or wherein m is 1 to about 50.

Oligomers of Formula I that are preferred are those wherein m is 1 to about 30, or m is 1 to about 25; more preferred are those wherein m is 1 to about 20, or those wherein m is 1 to about 10, or wherein m is 1 to about 5.

In some aspects of the invention, preferred oligomers are those oligomers of Formula I, or an acceptable salt or solvate thereof, wherein:

x is NR⁸, y is C=O, and R⁸ is hydrogen;
A₁ is optionally substituted *o*-, m-, or *p*-phenylene and A₂ is -(CH₂)_{q}-, wherein q is 1 or 2, and wherein A₁ and A₂ are independently optionally substituted with one or more polar (PL) group(s), one or more non-polar (NPL) group(s), or a combination of one or more polar (PL) group(s) and one or more non-polar (NPL) group(s); or
A₂ is optionally substituted *o*-, *m*-, or *p*-phenylene and A₁ is -(CH₂)_{q}-, wherein q is 1 or 2, and wherein A₁ and A₂ are independently optionally substituted with one or more polar (PL) group(s), one or more non-polar (NPL) group(s), or a combination of one or more polar (PL) group(s) and one or more non-polar (NPL) group(s);
R¹ and R² are independently hydrogen, a polar (PL) group, or a non-polar (NPL) group;
NPL is -(NR^{3'})_{q1NPL}-U^{NPL}-(CH₂)_{pNPL}-(NR^{3"})_{q2NPL}-R^{4'}, wherein:
R³, R^{3'}, and R^{3"} are independently selected from the group consisting of hydrogen, C₁-C₆ alkyl, and C₁-C₆ alkoxy;
R^{4'} is selected from the group consisting of hydrogen, alkyl, alkenyl, alkynyl, cycloalkyl, aryl, and heteroaryl, any of which is optionally substituted with one or more alkyl or halo groups;
U^{NPL} is absent or selected from the group consisting of O, S, S(=O), S(=O)₂, NR³, -C(=O)-, -C(=O)-N=N-NR³-, -C(=O)-NR³-N=N-, -N=N-NR³-, -C(=N-N(R³)₂)-, -C(=NR³)-, -C(=O)O-, -C(=O)S-, -C(=S)-, -O-P(=O)₂O-, -R³O-, -R³S-, -S-C=N- and -C(=O)-NR³-O- , wherein groups with two chemically nonequivalent termini can adopt both possible orientations;
the -(CH₂)_{pNPL}- alkylene chain is optionally substituted with one or more amino or hydroxy groups, or the alkylene chain is unsaturated;
pNPL is 0 to 8;
q1NPL and q2NPL are independently 0, 1 or 2;
PL is a polar group selected from the group consisting of halo, hydroxyethoxymethyl, methoxyethoxymethyl, polyoxyethylene, and -(NR^{5'})_{q1PL}-U^{PL}-(CH₂)_{pPL}-(NR^{5'})_{q2PL}-V, wherein:
R⁵, R^{5'}, and R^{5"} are independently selected from the group consisting of hydrogen, alkyl, and alkoxy;
U^{PL} is absent or selected from the group consisting of O, S, S(=O), S(=O)₂, NR⁵, -C(=O)-, -C(=O)-N=N-NR⁵-, -C(=O)-NR⁵-N=N-, -N=N-NR⁵-, -C(=N-N(R⁵)₂)-, -C(=NR⁵)-, -C(=O)O-, -C(=O)S-, -C(=S)-, -O-P(=O)₂O-, -R⁵O-, -R⁵S-, -S-C=N- and -C(=O)-NR⁵-O- , wherein groups with two chemically nonequivalent termini can adopt both possible orientations;
V is selected from the group consisting of nitro, cyano, amino, hydroxy, alkoxy, alkylthio, alkylamino, dialkylamino, -NH(CH₂)ₚNH₂ wherein p is 1 to 4, -N(CH₂CH₂NH₂)₂, diazamino, amidino, guanidino, guanyl, semicarbazone, aryl, heterocycle and heteroaryl, any of which is optionally substituted with one or more of amino, halo, cyano, nitro, hydroxy, -NH(CH₂)ₚNH₂ wherein p is 1 to 4,
-N(CH₂CH₂NH₂)₂, amidino, guanidino, guanyl, aminosulfonyl, aminoalkoxy, aminoalkythio, lower acylamino, or benzyloxycarbonyl;
the -(CH₂)_{pPL}- alkylene chain is optionally substituted with one or more amino or hydroxy groups, or the alkylene chain is unsaturated;
pPL is 0 to 8;
q1PL and q2PL are independently 0, 1 or 2; and
m is 4 or 5.

The polymers and oligomers of the present invention also include those of Formula II:

**R¹-[-x-A₁-x-y-A₂-y-]ₘ-R²** (II)

or an acceptable salt or solvate thereof,
wherein:
x is NR⁸, O, S, -N(R⁸)N(R⁸)-, -N(R⁸)-(N=N)-, -(N=N)-N(R⁸)-, -C(R⁷R^{7'})NR⁸-, -C(R⁷R^{7'})O-, or -C(R⁷R^{7'})S-; and y is C=O, C=S, O=S=O, - C(=O)C(=O)-, C(R⁶R^{6′})C=O or C(R⁶R^{6'})C=S; or
x and y are taken together to be pyromellitic diimide; wherein R⁸ is hydrogen or alkyl; R⁷ and R^{7'} are independently hydrogen or alkyl, or R⁷ and R^{7′} together are -(CH₂)ₚ-, wherein p is 4 to 8; and R⁶ and R^{6′} are independently hydrogen or alkyl, or R⁶ and R^{6'} together are (CH₂)₂NR¹²(CH₂)₂, wherein R¹² is hydrogen, -C(=N)CH₃ or C(=NH)-NH₂;
A₁ and A₂ are independently optionally substituted arylene or optionally substituted heteroarylene, wherein A₁ and A₂ are independently optionally substituted with one or more polar (PL) group(s), one or more non-polar (NPL) group(s), or a combination of one or more polar (PL) group(s) and one or more non-polar (NPL) group(s);
R¹ is
   (i) hydrogen, a polar group (PL), or a non-polar group (NPL), and R² is
      - x-A₁-x-R¹, wherein A₁ is as defined above and is optionally substituted with one or more polar (PL) group(s), one or more non-polar (NPL) group(s), or a combination of one or more polar (PL) group(s) and one or more non-polar (NPL) group(s); or
   (ii) hydrogen, a polar group (PL), or a non-polar group (NPL), and R² is -x-A′-x-R¹, wherein A' is aryl or heteroaryl and is optionally substituted with one or more polar (PL) group(s), one or more non-polar (NPL) group(s), or a combination of one or more polar (PL) group(s) and one or more non-polar (NPL) group(s);
   (iii) -y-A₂-y-R², and R² is hydrogen, a polar group (PL), or a non-polar group (NPL); or
   (iv) -y-A' and R² is -x-A', wherein A' is aryl or heteroaryl and is optionally substituted with one or more polar (PL) group(s), one or more non-polar (NPL) group(s), or a combination of one or more polar (PL) group(s) and one or more non-polar (NPL) group(s); or
   (v) R¹ and R² are independently a polar group (PL) or a non-polar group (NPL); or
   (vi) R¹ and R² together form a single bond;
NPL is a nonpolar group independently selected from the group consisting of -B(OR⁴)₂ and -(NR^{3′})_{q1NPL}-U^{NPL}-(CH₂)_{pNPL}-(NR^{3"})_{q2NPL}-R^{4′}, wherein:
   R³, R^{3′}, and R^{3"} are independently selected from the group consisting of hydrogen, alkyl, and alkoxy;
   R⁴ and R^{4'} are independently selected from the group consisting of hydrogen, alkyl, alkenyl, alkynyl, cycloalkyl, aryl, and heteroaryl, any of which is optionally substituted with one or more alkyl or halo groups;
   U^{NPL} is absent or selected from the group consisting of O, S, S(=O), S(=O)₂, NR³, -C(=O)-, -C(=O)-N=N-NR³-, -C(=O)-NR³-N=N-, -N=N-NR³-,-C(=N-N(R³)₂)-, -C(=NR³)-, -C(=O)O-, -C(=O)S-, -C(=S)-, -O-P(=O)₂O-, -R³O-, -R³S-, -S-C=N- and -C(=O)-NR³-O- , wherein groups with two chemically nonequivalent termini can adopt both possible orientations;
   the -(CH₂)_{pNPL}- alkylene chain is optionally substituted with one or more amino or hydroxy groups, or is unsaturated;
   pNPL is 0 to 8;
   q1NPL and q2NPL are independently 0, 1 or 2;
PL is a polar group selected from the group consisting of halo, hydroxyethoxymethyl, methoxyethoxymethyl, polyoxyethylene, and -(NR^{5'})_{q1PL}-U^{PL}-(CH₂)_{pPL}-(NR^{5"})_{q2PL}-V, wherein:
   R⁵, R^{5'}, and R^{5"} are independently selected from the group consisting of hydrogen, alkyl, and alkoxy;
   U^{PL} is absent or selected from the group consisting of O, S, S(=O), S(=O)₂, NR⁵, -C(=O)-, -C(=O)-N=N-NR⁵-, -C(=O)-NR⁵-N=N-, -N=N-NR⁵-, -C(=N-N(R⁵)₂)-, -C(=NR⁵)-, -C(=O)O-, -C(=O)S-, -C(=S)-, -O-P(=O)₂O-, -R⁵O-, -R⁵S-, -S-C=N- and -C(=O)-NR⁵-O- , wherein groups with two chemically nonequivalent termini can adopt both possible orientations;
   V is selected from the group consisting of nitro, cyano, amino, hydroxy, alkoxy, alkylthio, alkylamino, dialkylamino, -NH(CH₂)ₚNH₂ wherein p is 1 to 4, -N(CH₂CH₂NH₂)₂, diazamino, amidino, guanidino, guanyl, semicarbazone, aryl, heterocycle and heteroaryl, any of which is optionally substituted with one or more of amino, halo, cyano, nitro, hydroxy, -NH(CH₂)ₚNH₂ wherein p is 1 to 4, -N(CH₂CH₂NH₂)₂, amidino, guanidino, guanyl, aminosulfonyl, aminoalkoxy, aminoalkythio, lower acylamino, or benzyloxycarbonyl;
      the -(CH₂)_{pPL}- alkylene chain is optionally substituted with one or more amino or hydroxy groups, or is unsaturated;
      pPL is 0 to 8;
      q1PL and q2PL are independently 0,1 or 2; and
      m is 1 to about 500.

Polymers and oligomers of Formula II that are preferred for use in the disclosed method are those wherein x is NR⁸, O, S, or -N(R⁸)N(R⁸)-, and R⁸ is hydrogen or C₁-C₆ alkyl Also preferred are those polymers and oligomers wherein y is C=O, C=S, or O=S=O. Especially preferred are those polymers and oligomers wherein x is NR⁸ and y is C=O. For example, oligomers of Formula II wherein x is NH and y is C=O are especially preferred. Also preferred are oligomers of Formula II wherein x is O and y is C=O, or wherein x is -N(R⁸)N(R⁸)- and R⁸ is hydrogen, and y is C=O.

Preferred are those polymers and oligomers of Formula II wherein A₁ and A₂ are independently optionally substituted *o-*, *m-*, or *p*-phenylene. Those oligomers wherein A₁ and A₂ are optionally substituted *m*-phenylene are especially preferred. Also preferred are polymers and oligomers of Formula II wherein one of A₁ and A₂ is *o*-, *m*-, or *p*-phenylene, and the other of A₁ and A₂ is heteroarylene. Preferred heteroarylene groups include, but are not limited to, pyridinylene, pyrimidinylene, and pyrazinylene.

Also preferred are polymers and oligomers of Formula II wherein A₁ and A₂ are independently optionally substituted arylene or optionally substituted heteroarylene, and (i) one of A₁ and A₂ is substituted with one or more polar (PL) group(s) and one or more nonpolar (NPL) group(s) and the other of A₁ and A₂ is unsubstituted; or (ii) one of A₁ and A₂ is substituted with one or more polar (PL) group(s) and one or more nonpolar (NPL) group(s) and the other of A₁ and A₂ is substituted with one or more polar (PL) group(s). Polymers and oligomers in which either (i) one of A₁ and A₂ is substituted with one polar (PL) group and one nonpolar (NPL) group, and the other of A₁ and A₂ is unsubstituted, or (ii) one of A₁ and A₂ is substituted with one polar (PL) group and one nonpolar (NPL) group and the other of A₁ and A₂ is substituted with one or two polar (PL) group(s), are especially preferred.

Thus, polymers and oligomers of Formula II are preferred in which A₁ and A₂ are optionally substituted *m*-phenylene, wherein one of A₁ and A₂ is substituted with one polar (PL) group and one nonpolar (NPL) group and the other of A₁ and A₂ is unsubstituted.

In some aspects of the invention, preferred polymers and oligomers of Formula II are those wherein (i) R¹ is hydrogen, a polar group (PL), or a non-polar group (NPL), and R² is -x-A₁-x-R¹, wherein A₁ is as defined above and is substituted with one or more polar (PL) group(s), one or more non-polar (NPL) group(s), or a combination of one or more polar (PL) group(s) and one or more non-polar (NPL) group(s), or (ii) R¹ is hydrogen, a polar group (PL), or a non-polar group (NPL), and R² is -x-A'-x-R¹, wherein A' is aryl or heteroaryl and is optionally substituted with one or more polar (PL) group(s), one or more non-polar (NPL) group(s), or a combination of one or more polar (PL) group(s) and one or more non-polar (NPL) group(s).

More preferred are those oligomers of Formula II wherein R¹ is hydrogen, a polar group (PL), or a non-polar group (NPL), and R² is -x-A₁-x-R¹, wherein A₁ is as defined above and is substituted with one or more polar (PL) group(s), one or more non-polar (NPL) group(s), or a combination of one or more polar (PL) group(s) and one or more non-polar (NPL) group(s). More preferred still are those oligomers wherein R¹ is hydrogen or a polar group (PL), and R² is -x-A₁-x-R¹, where A₁ is substituted with one or more polar (PL) group(s) or a combination of one or more polar (PL) group(s) and one or more non-polar (NPL) group(s). Especially preferred are those oligomers wherein R¹ is a polar (PL) group and R² is -x-A₁-x-R¹, where A₁ is substituted with one or two polar (PL) group(s) and one non-polar (NPL) group.

In some aspects of the invention, preferred polymers and oligomers of Formula II are those wherein NPL is -B(OR⁴)₂. Preferred values of R⁴ are hydrogen, C₁-C₁₀ alkyl, C₃-C₁₈ branched alkyl, C₂-C₁₀ alkenyl, C₂-C₁₀ alkynyl, C₃-C₈ cycloalkyl, C₆-C₁₀ aryl, and heteroaryl, any of which is optionally substituted with one or more C₁-C₆ alkyl or halo groups.

In other aspects, preferred polymers and oligomers of Formula II include those wherein NPL is -(NR^{3'})_{q1NPL}-U^{NPL}-(CH₂)_{pNPL}-(NR^{3"})_{q2NPL}-R^{4'}, and R³, R^{3′}, R^{3"}, R^{4'}, U^{NPL}, pNPL, q1NPL and q2NPL are as defined above.

Preferred values for each of R³, R^{3'}, and R^{3"} are hydrogen, C₁-C₆ alkyl, and C₁-C₆ alkoxy. Hydrogen is an especially preferred value for R³, R^{3′}, and R^{3"}.

Preferred values of R^{4′} are hydrogen, C₁-C₁₀ alkyl, C₃-C₁₈ branched alkyl, C₂-C₁₀ alkenyl, C₂-C₁₀ alkynyl, C₃-C₈ cycloalkyl, C₆-C₁₀ aryl, and heteroaryl, any of which is optionally substituted with one or more C₁-C₆ alkyl or halo groups. Values of R^{4′} that are more preferred are C₁-C₁₀ alkyl, C₃-C₁₈ branched alkyl, C₂-C₁₀ alkenyl, C₂-C₁₀ alkynyl, and C₆-C₁₀ aryl, especially phenyl. Especially preferred values of R^{4′} are C₁-C₁₀ alkyl and C₃-C₁₈ branched alkyl. Suitable C₁-C₁₀ alkyl and C₃-C₁₈ branched alkyl groups are methyl, ethyl, *n*-propyl, isopropyl, *n*-butyl, *tert*-butyl, and *n*-pentyl.

Preferred values of U^{NPL} are O, S, S(=O), S(=O)₂, NH, -C(=O)-, -C(=O)-N=N-NH-, -C(=O)-NH-N=N-, -N=N-NH-, -C(=N-N(R³)₂)-, -C(=NR³)-, -C(=O)O-, -C(=O)S-, -C(=S)-, -O-P(=O)₂O-, -R³O-, -R³S-, -S-C=N- and -C(=O)-NR³-O-, wherein groups with two chemically nonequivalent termini can adopt both possible orientations.

Values of U^{NPL} that are more preferred are O, S, NH, -C(=O)-, -C(=O)-N=N-NH-, -C(=O)-NH-N=N-, -N=N-NH-, -C(=N-N(R³)₂)-, -C(=NR³)-, -C(=O)O-, -R³S- and -R³O-. An especially preferred value of U^{NPL} is -C(=O)-. Preferred polymers and oligomers of Formula II also include those wherein U^{NPL} is absent.

In some aspects of the invention, preferred polymers and oligomers of Formula II are those wherein U^{NPL} is -O-P(=O)₂O-.

Preferred values of pNPL are 0 to 6; values of pNPL of 0 to 4 are especially preferred, with values of pNPL of 0, 1 or 2 most preferred.

Preferred values of q1NPL and q2NPL are 0 or 1. Values of q1NPL and q2NPL of 0 or 1 are especially preferred, with a value of 0 being the most preferred for each of q1NPL and q2NPL.

In preferred polymers and oligomers of Formula II, the -(CH₂)_{pNPL}-alkylene chain in NPL is unsubstituted or substituted with one or more amino or hydroxy groups. More preferred are those oligomers of Formula II wherein the -(CH₂)_{pNPL}- alkylene chain in NPL is substituted with one or more amino groups.

An especially preferred value of NPL for polymers and oligomers of Formula II is C₁-C₆ alkyl. Examples of preferred values for NPL are *n*-propyl, isopropyl, *n*-butyl, and *tert*-butyl.

In some aspects of the invention, preferred polymers and oligomers of Formula II are those wherein PL is -(NR^{5'})_{q1PL}-U^{PL}-(CH₂)_{pPL}-(NR^{5"})_{q2PL} -V, and R⁵, R^{5'}, R^{5"}, V, U^{PL}, pPL, q1PL and q2PL are as defined above.

Preferred values for R⁵, R^{5'}, and R^{5"} are hydrogen, C₁-C₆ alkyl, and C₁-C₆ alkoxy. Hydrogen is an especially preferred value for each of R⁵, R^{5'}, and R^{5"}.

Preferred values of U^{PL} are O, S, S(=O), S(=O)₂, NH, -C(=O)-, -C(=O)-N=N-NH-, -C(=O)-NH-N=N-, -N=N-NH-, -C(=N-N(R⁵)₂)-, -C(=NR⁵)-, -C(=O)O-, -C(=O)S-, -C(=S)-, -O-P(=O)₂O-, -R⁵O-, -R⁵S-, -S-C=N- and -C(=O)-NR⁵-O-, wherein groups with two chemically nonequivalent termini can adopt both possible orientations. Values of U^{PL} that are more preferred are O, S, NH, -C(=O)-, -C(=O)-N=N-NH-, -C(=O)-NH-N=N-, -N=N-NH-, -C(=N-N(R⁵)₂)-, -C(=NR⁵)-, -C(=O)O-, -R⁵Sand -R⁵O-. Especially preferred values of U^{PL} are O, S, and -C(=O). Preferred polymers and oligomers of Formula II are also those wherein U^{PL} is absent.

In some aspects of the invention, preferred polymers and oligomers of Formula II are those wherein U^{PL} is -O-P(=O)₂O-.

Preferred values of V are nitro, cyano, amino, hydroxy, C₁-C₆ alkoxy, C₁-C₆ alkylthio, C₁-C₆ alkylamino, C₁-C₆ dialkylamino, -NH(CH₂)ₚNH₂ wherein p is 1 to 4, -N(CH₂CH₂NH₂)₂, diazamino, amidino, guanidino, guanyl, semicarbazone, C₆-C₁₀ aryl, heterocycle, and heteroaryl, any of which is optionally substituted with one or more of amino, halo, cyano, nitro, hydroxy, -NH(CH₂)ₚNH₂ wherein p is 1 to 4, -N(CH₂CH₂NH₂)₂, amidino, guanidino, guanyl, aminosulfonyl, aminoalkoxy, lower acylamino, or benzyloxycarbonyl.

Suitable heteroaryl groups include indolyl, 3*H*-indolyl, 1*H-*isoindolyl, indazolyl, benzoxazolyl, pyridyl, and 2-aminopyridyl. Suitable heterocycle groups include piperidinyl, piperazinyl, imidazolidinyl, pyrrolidinyl, pyrazolidinyl, and morpholinyl.

Values of V that are more preferred are amino, C₁-C₆ alkylamino, C₁-C₆ dialkylamino, -NH(CH₂)ₚNH₂ wherein p is 1 to 4, -N(CH₂CH₂NH₂)₂, diazamino, amidino, guanidino, guanyl, and semicarbazone, preferably any of which is optionally substituted with one or more of amino, halo, cyano, nitro, hydroxy, -NH(CH₂)ₚNH₂ wherein p is 1 to 4, -N(CH₂CH₂NH₂)₂, amidino, guanidino, guanyl, aminosulfonyl, aminoalkoxy, lower acylamino, or benzyloxycarbonyl.

Especially preferred values of V are amino, C₁-C₆ alkylamino, -NH(CH₂)ₚNH₂ wherein p is 1 to 4, -N(CH₂CH₂NH₂)₂, diazamino, amidino, and guanidino, preferably any of which is optionally substituted with one or more of amino, halo, cyano, nitro, hydroxy, -NH(CH₂)ₚNH₂ wherein p is 1 to 4, -N(CH₂CH₂NH₂)₂, amidino, guanyl, guanidino, or aminoalkoxy. Values of V that are most preferred are amino and guanidino.

Preferred values of pPL are 0 to 6; values of pPL of 0 to 4 are especially preferred, with values of pPL of 2 to 4 especially preferred.

Preferred values of q1PL and q2PL are 0 or 1. Values of q1PL and q2PL of 0 or 1 are especially preferred, with a value of 0 being especially preferred for each of q1PL and q2PL.

In preferred polymers and oligomers of Formula II, the -(CH₂)_{pPL}-alkylene chain in PL is optionally substituted with one or more amino or hydroxy groups.

Preferred polymers of Formula II are those in which m is 1 to about 500. Especially preferred are those polymers of Formula II wherein m is 1 to about 100, or wherein m is 1 to about 50.

Oligomers of Formula II that are preferred are those wherein m is 1 to about 30, or m is 1 to about 25; more preferred are those wherein m is 1 to about 20, or wherein m is 1 to about 10, or wherein m is 1 to about 5. Especially preferred are those oligomers of Formula II wherein m is 1, 2 or 3.

In some aspects, preferred oligomers of Formula II are those wherein:
x is NR⁸, y is C=O, and R⁸ is hydrogen or alkyl;
A₁ and A₂ are independently optionally substituted *o*-, *m*-, or *p*-phenylene or pyrimidinylene, wherein A₁ and A₂ are independently optionally substituted with one or more polar (PL) group(s), one or more non-polar (NPL) group(s), or a combination of one or more polar (PL) group(s) and one or more non-polar (NPL) group(s);
R¹ is hydrogen, a polar group (PL), or a non-polar group (NPL), and R² is -x-A₁-x-R¹, wherein A₁ is as defined above and is optionally substituted with one or more polar (PL) group(s), one or more non-polar (NPL) group(s), or a combination of one or more polar (PL) group(s) and one or more non-polar (NPL) group(s);
NPL is -(NR^{3′})_{q1NPL}-U^{NPL}-(CH₂)_{pNPL}-(NR^{3"})_{q2NPL}-R^{4'}, wherein:
   R³, R^{3'}, and R^{3"} are independently selected from the group consisting of hydrogen, C₁-C₆ alkyl, and C₁-C₆ alkoxy;
   R^{4'} is selected from the group consisting of hydrogen, C₁-C₁₀ alkyl, C₃-C₁₈ branched alkyl, C₂-C₁₀ alkenyl, C₂-C₁₀ alkynyl, C₃-C₈ cycloalkyl, C₆-C₁₀ aryl, and heteroaryl, any of which is optionally substituted with one or more C₁-C₆ alkyl or halo groups;
   U^{NPL} is absent or selected from the group consisting of O, S, NH, -C(=O)-, -C(=O)-N=N-NH-, -C(=O)-NH-N=N-, -N=N-NH-, -C(=N-N(R³)₂)-, -C(=NR³)-, -C(=O)O-, -R³S- and -R³O-, wherein groups with two chemically nonequivalent termini can adopt both possible orientations;
the -(CH₂)_{pNPL}- alkylene chain is optionally substituted with one or more amino or hydroxy groups;
pNPL is 0 to 6;
q1NPL and q2NPL are independently 0;
PL is -(NR^{5'})_{q1PL}-U^{PL}-(CH₂)_{pPL}-(NR^{5"})_{q2PL}-V, wherein:
R⁵, R^{5'}, and R^{5"} are independently selected from the group consisting of hydrogen, C₁-C₆ alkyl, and C₁-C₆ alkoxy;
U^{PL} is absent or selected from the group consisting of O, S, NH, - C(=O)-, -C(=O)-N=N-NH-, -C(=O)-NH-N=N-, -N=N-NH-, -C(=N-N(R⁵)₂)-, -C(=NR⁵)-, -C(=O)O-, -R⁵O-, and -R⁵S-, wherein groups with two chemically nonequivalent termini can adopt both possible orientations;
V is selected from the group consisting of nitro, cyano, amino, hydroxy, C₁-C₆ alkoxy, C₁-C₆ alkylthio, C₁-C₆ alkylamino, C₁-C₆ dialkylamino, -NH(CH₂)ₚNH₂ wherein p is 1 to 4, -N(CH₂CH₂NH₂)₂, diazamino, amidino, guanidino, guanyl, semicarbazone, C₆-C₁₀ aryl, heterocycle, and heteroaryl, preferably any of which is optionally substituted with one or more of amino, halo, cyano, nitro, hydroxy, -NH(CH₂)ₚNH₂ wherein p is 1 to 4, -N(CH₂CH₂NH₂)₂, amidino, guanidino, guanyl, aminosulfonyl, aminoalkoxy, lower acylamino, or benzyloxycarbonyl;
the -(CH₂)_{pPL}- alkylene chain is optionally substituted with one or more amino or hydroxy groups;
pPL is 0 to 6;
q1PL and q2PL are 0; and
m is1 to10.

Preferred oligomers of the invention also include an oligomer of Formula IIa:

**R¹-x-A₁-x-y-A₂-y-x-A₁-x-R²** (IIa)

or an acceptable salt or solvate thereof,
wherein:
x is NR⁸, O, S, or -N(R⁸)N(R⁸)-; and y is C=O, C=S, or O=S=O; wherein R⁸ is hydrogen or alkyl;
A₁ and A₂ are independently optionally substituted arylene or optionally substituted heteroarylene, wherein A₁ and A₂ are independently optionally substituted with one or more polar (PL) group(s), one or more non-polar (NPL) group(s), or a combination of one or more polar (PL) group(s) and one or more non-polar (NPL) group(s);
R¹ is a polar group (PL) or a non-polar group (NPL); and R² is R¹;
NPL is a nonpolar group independently selected from the group consisting of -B(OR⁴)₂ and -(NR^{3'})_{q1NPL}-U^{NPL}-(CH₂)_{pNPL}-(NR^{3"})_{q2NPL}-R^{4'}, wherein:
   R³, R^{3'}, and R^{3"} are independently selected from the group consisting of hydrogen, alkyl, and alkoxy;
   R⁴ and R^{4'} are independently selected from the group consisting of hydrogen, alkyl, alkenyl, alkynyl, cycloalkyl, aryl, and heteroaryl, any of which is optionally substituted with one or more alkyl or halo groups;
   U^{NPL} is absent or selected from the group consisting of O, S, S(=O), S(=O)₂, NR³, -C(=O)-, -C(=O)-N=N-NR³-, -C(=O)-NR³-N=N-, -N=N-NR³-, -C(=N-N(R³)₂)-, -C(=NR³)-, -C(=O)O-, -C(=O)S-, -C(=S)-, -O-P(=O)₂O-, -R³O-, -R³S-, -S-C=N- and -C(=O)-NR³-O- , wherein groups with two chemically nonequivalent termini can adopt both possible orientations;
   the -(CH₂)_{pNPL}- alkylene chain is optionally substituted with one or more amino or hydroxy groups, or is unsaturated;
   pNPL is 0 to 8;
   q1NPL and q2NPL are independently 0, 1 or 2;
PL is a polar group selected from the group consisting of halo, hydroxyethoxymethyl, methoxyethoxymethyl, polyoxyethylene, and -(NR^{5'})_{q1PL}-U^{PL}-(CH₂)_{pPL}-(NR^{5"})_{q2PL}-V, wherein:
   R⁵, R^{5'}, and R^{5"} are independently selected from the group consisting of hydrogen, alkyl, and alkoxy;
   U^{PL} is absent or selected from the group consisting of O, S, S(=O), S(=O)₂, NR⁵, -C(=O)-, -C(=O)-N=N-NR⁵-, -C(=O)-NR⁵-N=N-, -N=N-NR⁵-, -C(=N-N(R⁵)₂)-, -C(=NR⁵)-, -C(=O)O-, -C(=O)S-, -C(=S)-,-O-P(=O)₂O-, -R⁵O-, -R⁵S-, -S-C=N- and -C(=O)-NR⁵-O- , wherein groups with two chemically nonequivalent termini can adopt both possible orientations;
   V is selected from the group consisting of nitro, cyano, amino, hydroxy, alkoxy, alkylthio, alkylamino, dialkylamino, -NH(CH₂)ₚNH₂ wherein p is 1 to 4, -N(CH₂CH₂NH₂)₂, diazamino, amidino, guanidino, guanyl, semicarbazone, aryl, heterocycle and heteroaryl, preferably any of which is optionally substituted with one or more of amino, halo, cyano, nitro, hydroxy, -NH(CH₂)ₚNH₂ wherein p is 1 to 4, -N(CH₂CH₂NH₂)₂, amidino, guanidino, guanyl, aminosulfonyl, aminoalkoxy, aminoalkythio, lower acylamino, or benzyloxycarbonyl;
   the -(CH₂)_{pPL}- alkylene chain is optionally substituted with one or more amino or hydroxy groups, or is unsaturated;
   pPL is 0 to 8; and
   q1PL and q2PL are independently 0, 1 or 2;
and a pharmaceutically acceptable carrier or diluent.

Preferred oligomers of Formula IIa are those wherein x is NR⁸ and y is C=O. For example, oligomers of Formula IIa wherein x is NH and y is C=O are especially preferred. Also preferred are oligomers of Formula IIa wherein x is O and y is C=O, or wherein x is -N(R⁸)N(R⁸)- and R⁸ is hydrogen, and y is C=O.

Preferred are those oligomers of Formula IIa wherein A₁ and A₂ are independently optionally substituted *o*-, *m-,* or *p*-phenylene, especially *m*— phenylene. Also preferred are oligomers of Formula IIa wherein one of A₁ and A₂ is *o*-, *m*-, or *p*-phenylene, and the other of A₁ and A₂ is heteroarylene. Preferred heteroarylene groups include, but are not limited to, pyridinylene, pyrimidinylene, and pyrazinylene.

As for the polymers and oligomers of Formula II, preferred oligomers of Formula IIa are those wherein A₁ and A₂ are independently optionally substituted arylene or optionally substituted heteroarylene, and (i) one of A₁ and A₂ is substituted with one or more polar (PL) group(s) and one or more nonpolar (NPL) group(s) and the other of A₁ and A₂ is unsubstituted; or (ii) one of A₁ and A₂ is substituted with one or more polar (PL) group(s) and one or more nonpolar (NPL) group(s) and the other of A₁ and A₂ is substituted with one or more polar (PL) group(s). Especially preferred are oligomers in which either (i) one of A₁ and A₂ is substituted with one polar (PL) group and one nonpolar (NPL) group, and the other of A₁ and A₂ is unsubstituted, or (ii) one of A₁ and A₂ is substituted with one polar (PL) group and one nonpolar (NPL) group and the other of A₁ and A₂ is substituted with one or two polar (PL) group(s), are especially preferred.

Preferred oligomers of Formula IIa are those in which R¹ and R² are either hydrogen or a polar (PL) group.

Preferred values of R³, R^{3'}, R^{3"}, R⁴, R^{4'}, NPL, U^{NPL}, pNPL, q1NPL, q2NPL, PL, R⁵, R^{5'}, R^{5"}, V, U^{PL}, pPL, q1PL and q2PL for the oligomers of Formula IIa are also the same as those listed for polymers and oligomers of Formula II above.

Polymers and oligomers of the present invention also include those of Formula IV:

**R1-[-x-A₁-x-z-y-A₂-y-z]ₘ-R²** (IV)

or an acceptable salt or solvate thereof,
wherein:
x is NR⁸, -NR⁸NR⁸-, C=O, or O; y is NR⁸, -NR⁸NR⁸-, C=O, S, or O; and R⁸ is hydrogen or alkyl;
z is C=O, C=S, O=S=O, -NR⁸NR⁸-, or -C(=O)C(=O)- ;
A₁ and A₂ are independently optionally substituted arylene or optionally substituted heteroarylene, wherein A₁ and A₂ are independently optionally substituted with one or more polar (PL) group(s), one or more non-polar (NPL) group(s), or a combination of one or more polar (PL) group(s) and one or more non-polar (NPL) group(s);
R¹ is
   (i) hydrogen, a polar group (PL), or a non-polar group (NPL), and R² is -x-A₁-x-R¹ wherein A₁ is as defined above and is optionally substituted with one or more polar (PL) group(s), one or more non-polar (NPL) group(s), or a combination of one or more polar (PL) group(s) and one or more non-polar (NPL) group(s); or
   (ii) hydrogen, a polar group (PL), or a non-polar group (NPL), and R² is -x-A₁-x-z-y-A₂-y-R¹, wherein A₁ and A₂ are as defined above, and each of which is optionally substituted with one or more polar (PL) group(s), one or more non-polar (NPL) group(s), or a combination of one or more polar (PL) group(s) and one or more non-polar (NPL) group(s); or
   (iii) hydrogen, a polar group (PL), or a non-polar group (NPL), and R² is -x-A^{'}-x-R¹, wherein A^{'} is aryl or heteroaryl and is optionally substituted with one or more polar (PL) group(s), one or more non-polar (NPL) group(s), or a combination of one or more polar (PL) group(s) and one or more non-polar (NPL) group(s); or
   (iv) hydrogen, a polar group (PL), or a non-polar group (NPL), and R² is -x-A₁-x-z-y-A^{'}-y-R¹, wherein A₁ is as defined above, A^{'} is aryl or heteroaryl, and each of A₁ and A^{'} is optionally substituted with one or more polar (PL) group(s), one or more non-polar (NPL) group(s), or a combination of one or more polar (PL) group(s) and one or more non-polar (NPL) group(s); or
   (v) -z-y-A^{'} and R² is hydrogen, a polar group (PL), or a non-polar group (NPL), wherein A' is aryl or heteroaryl and is optionally substituted with one or more polar (PL) group(s), one or more non-polar (NPL) group(s), or a combination of one or more polar (PL) group(s) and one or more non-polar (NPL) group(s); or
   (vi) z-y-A', and R² is -x-A^{"} wherein A^{'} and A^{"} are independently aryl or heteroaryl, and each of A^{'} and A^{"} is optionally substituted with one or more polar (PL) group(s), one or more non-polar (NPL) group(s), or a combination of one or more polar (PL) group(s) and one or more non-polar (NPL) group(s); or
   (vii) R¹ and R² are independently a polar group (PL) or a non-polar group (NPL); or
   (viii) R¹ and R² together form a single bond;
NPL is a nonpolar group independently selected from the group consisting of
   -B(OR⁴)₂ and -(NR^{3′})_{q1NPL}-U^{NPL}-(CH₂)_{pNPL}-(NR^{3"})_{q2NPL}-R^{4'}, wherein: of R³, R^{3'} and R^{3"} are independently selected from the group consisting of hydrogen, alkyl, and alkoxy;
   R⁴ and R^{4'} are independently selected from the group consisting of hydrogen, alkyl, alkenyl, alkynyl, cycloalkyl, aryl, and heteroaryl, any of which is optionally substituted with one or more alkyl or halo groups;
   U^{NPL} is absent or selected from the group consisting of O, S, S(=O), S(=O)₂, NR³, -C(=O)-, -C(=O)-N=N-NR³-, -C(=O)-NR³-N=N-, -N=N-NR³-, -C(=N-N(R³)₂)-, -C(=NR³)-, -C(=O)O-, -C(=O)S-, -C(=S)-, -O-P(=O)₂O-, -R³O-, -R³S-, -S-C=N- and -C(=O)-NR³-O- , wherein groups with two chemically nonequivalent termini can adopt both possible orientations;
the -(CH₂)_{pNPL}- alkylene chain is optionally substituted with one or more amino or hydroxy groups, or is unsaturated;
pNPL is 0 to 8;
q1NPL and q2NPL are independently 0, 1 or 2;
   PL is a polar group selected from the group consisting of halo, hydroxyethoxymethyl, methoxyethoxymethyl, polyoxyethylene, and -(NR^{5'}_{q1PL}-U^{PL}-(CH₂)_{pPL}-(NR^{5"}_{q2PL}-V, wherein:
   R⁵, R^{5'} and R^{5"}are independently selected from the group consisting of hydrogen, alkyl, and alkoxy;
   U^{PL} is absent or selected from the group consisting of O, S, S(=O), S(=O)₂, NR⁵, -C(=O)-, -C(=O)-N=N-NR⁵-, -C(=O)-NR⁵-N=N-, -N=N-NR⁵-, -C(=N-N(R⁵)₂)-, -C(=NR⁵)-, -C(=O)O-, -C(=O)S-, -C(=S)-, -O-P(=O)₂O-, -R⁵O-, -R⁵S-, -S-C=N- and -C(=O)-NR⁵-O- , wherein groups with two chemically nonequivalent termini can adopt both possible orientations;
   V is selected from the group consisting of nitro, cyano, amino, hydroxy, alkoxy, alkylthio, alkylamino, dialkylamino, -NH(CH₂)ₚNH₂ wherein p is 1 to 4, -N(CH₂CH₂NH₂)₂, diazamino, amidino, guanidino, guanyl, semicarbazone, aryl, heterocycle and heteroaryl, any of which is optionally substituted with one or more of amino, halo, cyano, nitro, hydroxy, -NH(CH₂)ₚNH₂ wherein p is 1 to 4, -N(CH₂CH₂NH₂)₂, amidino, guanidino, guanyl, aminosulfonyl, aminoalkoxy, aminoalkythio, lower acylamino, or benzyloxycarbonyl;
   the -(CH₂)_{pPL}- alkylene chain is optionally substituted with one or more amino or hydroxy groups, or is unsaturated;
   pPL is 0 to 8;
   q1PL and q2PL are independently 0, 1 or 2; and
m is 1 to about 500.

Polymers and oligomers of Formula IV that are preferred for use in the disclosed method are those wherein x and y are independently NR⁸, C=O, or O; z is C=O or -NR⁸NR⁸; and R⁸ is hydrogen or C₁-C₆ alkyl. Especially preferred are those polymers and oligomers wherein x and y are each NR⁸, z is C=O, and R⁸ is hydrogen. Also preferred are oligomers of Formula IV wherein x is NR⁸ or O, y is O, and z is C=O, or wherein x and y are each C=O, and z is -N(R⁸)N(R⁸)-, especially wherein R⁸ is hydrogen.

Preferred are those polymers and oligomers of Formula IV wherein A₁ and A₂ are independently optionally substituted *o*-, *m-,* or *p*-phenylene. Those oligomers wherein A₁ and A₂ are optionally substituted *m*-henylene are especially preferred. Also preferred are polymers and oligomers of Formula IV wherein one of A₁ and A₂ is *o*-, *m-,* or *p*-phenylene, and the other of A₁ and A₂ is heteroarylene. Preferred heteroarylene groups include, but are not limited to, pyridinylene, pyrimidinylene, and pyrazinylene.

Also preferred are polymers and oligomers of Formula IV wherein A₁ and A₂ are independently optionally substituted arylene or optionally substituted heteroarylene, and (i) each of A₁ and A₂ is substituted with one or more polar (PL) group(s) and one or more nonpolar (NPL) group(s); or (ii) one of A₁ and A₂ is substituted with one or more polar (PL) group(s) and the other of A₁ and A₂ is substituted with one or more nonpolar (NPL) group(s). Polymers and oligomers in which (i) each of A₁ and A₂ is substituted with one polar (PL) group and one nonpolar (NPL) group, or (ii) one of A₁ and A₂ is substituted with one or two polar (PL) group(s) and the other of A₁ and A₂ is substituted with one or two nonpolar (NPL) group(s), are especially preferred.

In some aspects of the invention, preferred polymers and oligomers of Formula IV are those wherein (i) R¹ is hydrogen, a polar group (PL), or a non-polar group (NPL), and R² is -x-A₁-x-R¹, wherein A₁ is as defined above and is optionally substituted with one or more polar (PL) group(s), one or more nonpolar (NPL) group(s), or a combination of one or more polar (PL) group(s) and one or more non-polar (NPL) group(s); or (iii) R¹ is hydrogen, a polar group (PL), or a non-polar group (NPL), and R² is -x-A₁-x-z-y-A₂-y-R¹, wherein A₁ and A₂ are as defined above, and each of which is optionally substituted with one or more polar (PL) group(s), one or more nonpolar (NPL) group(s), or a combination of one or more polar (PL) group(s) and one or more non-polar (NPL) group(s).

More preferred are those oligomers of Formula IV wherein R¹ is hydrogen, a polar group (PL), or a non-polar group (NPL), and R² is -x-A₁-x-R¹, wherein A₁ is as defined above and is substituted with one polar (PL) group and one non-polar (NPL) group; or those oligomers wherein R¹ is hydrogen, a polar group (PL), or a non-polar group (NPL), and R² is -x-A₁-x-z-y-A₂-y-R¹, wherein A₁ and A₂ are as defined above, and each of which is substituted with one polar (PL) group and one non-polar (NPL) group.

In some aspects, preferred polymers and oligomers of Formula IV are those wherein NPL is ―(NR^{3'}_{q1NPL}-U^{NPL}-(CH₂)_{pNPL}-(NR^{3"}_{q2NPL}-R^{4'} and R³, R^{3'} R^{3"} R^{4'} U^{NPL}, pNPL, q1NPL and q2NPL are as defined above.

Preferred values for each of R³, R^{3'} and R^{3"}are hydrogen, C₁-C₆ alkyl, and C₁-C₆ alkoxy. Hydrogen is an especially preferred value for R³, R^{3'} , and R^{3"}.

Preferred values of R^{4'} are hydrogen, C₁-C₁₀ alkyl, C₃-C₁₈ branched alkyl, C₂-C₁₀ alkenyl, C₂-C₁₀ alkynyl, C₃-C₈ cycloalkyl, C₆-C₁₀ aryl, and heteroaryl, any of which is optionally substituted with one or more C₁-C₆ alkyl or halo groups. Values of R^{4'} that are more preferred are C₁-C₁₀ alkyl, C₃-C₁₈ branched alkyl, C₂-C₁₀ alkenyl, C₂-C₁₀ alkynyl, and C₆-C₁₀ aryl, especially phenyl. Especially preferred values of R^{4'} are C₁-C₁₀ alkyl and C₃-C₁₈ branched alkyl. Suitable C₁-C₁₀ alkyl and C₃-C₁₈ branched alkyl groups are methyl, ethyl, *n*-propyl, isopropyl, *n*-butyl, *tert*-butyl, and *n*-pentyl.

Preferred values of U^{NPL} are O, S, NH, -C(=O)-, -C(=O)-N=N-NH-, -C(=O)-NH-N=N-, -N=N-NH-, -C(=N-N(R³)₂)-, -C(=NR³)-, -C(=O)O-, -R³Sand -R³O-.

Especially preferred polymers and oligomers of Formula IV are those wherein U^{NPL} is absent.

Preferred values of pNPL are 0 to 6; values of pNPL of 0 to 4 are especially preferred, with values of pNPL of 0, 1 or 2 most preferred.

Preferred values of q1NPL and q2NPL are 0 or 1. Values of q1NPL and q2NPL of 0 or 1 are especially preferred, with a value of 0 being the most preferred for each of q1NPL and q2NPL.

In preferred polymers and oligomers of Formula IV, the -(CH₂)_{pNPL}-alkylene chain in NPL is unsubstituted or substituted with one or more amino or hydroxy groups. More preferred are those oligomers of Formula IV wherein the -(CH₂)_{pNPL}- alkylene chain in NPL is substituted with one or more amino groups.

An especially preferred value of NPL for polymers and oligomers of Formula IV is C₁-C₆ alkyl. Examples of preferred values for NPL are n-propyl, isopropyl, n-butyl, and tert-butyl.

In some aspects of the invention, preferred polymers and oligomers of Formula IV are those wherein PL is -(NR^{5'})_{q1PL}-U^{PL}-(CH₂)_{pPL-}(NR^{5"})_{q2PL} -V, and R⁵, R^{5'}, R^{5"}, V, U^{PL}, pPL, q1PL and q2PL are as defined above.

Preferred values for R⁵, R^{5'}, and R^{5"} are hydrogen, C₁-C₆ alkyl, and C₁-C₆ alkoxy. Hydrogen is an especially preferred value for each of R⁵, R^{5'}, and R^{5"}_{.}

Preferred values of U^{PL} are O, S, NH, -C(=O)-, -C(=O)-N=N-NH-, -C(=O)-NH-N=N-, -N=N-NH-, -C(=N-N(R⁵)₂)-, -C(=NR⁵)-, -C(=O)O-, -R⁵Sand -R⁵O-. Especially preferred values of U^{PL} are O, S, and -C(=O).

In some aspects of the invention, preferred polymers and oligomers of Formula IV are those wherein U^{PL} is -O-P(=O)₂O-.

Preferred values of V are nitro, cyano, amino, 1, C₁-C₆ alkoxy, C₁-C₆ alkylthio, C₁-C₆ alkylamino, C₁-C₆ dialkylamino, -NH(CH₂)pNH₂ wherein p is 1 to 4, -N(CH₂CH₂NH₂)₂, diazamino, amidino, guanidino, guanyl, semicarbazone, C₆-C₁₀ aryl, heterocycle, and heteroaryl, preferably any of which is optionally substituted with one or more of amino, halo, cyano, nitro, hydroxy, -NH(CH₂)ₚNH₂ wherein p is 1 to 4, -N(CH₂CH₂NH₂)₂, amidino, guanidino, guanyl, aminosulfonyl, aminoalkoxy, lower acylamino, or benzyloxycarbonyl.

Suitable heteroaryl groups include indolyl, 3*H*-indolyl, 1*H*-isoindolyl, indazolyl, benzoxazolyl, pyridyl, and 2-aminopyridyl. Suitable heterocycle groups include piperidinyl, piperazinyl, imidazolidinyl, pyrrolidinyl, pyrazolidinyl, and morpholinyl.

Values of V that are more preferred are amino, C₁-C₆ alkylamino, C₁-C₆ dialkylamino, -NH(CH₂)pNH₂ wherein p is 1 to 4, -N(CH₂CH₂NH₂)₂, diazamino, amidino, guanidino, guanyl, and semicarbazone, preferably any of which is optionally substituted with one or more of amino, halo, cyano, nitro, hydroxy, -NH(CH₂)ₚNH₂ wherein p is 1 to 4, -N(CH₂CH₂NH₂)₂, amidino, guanidino, guanyl, aminosulfonyl, aminoalkoxy, lower acylamino, or benzyloxycarbonyl.

Especially preferred values of V are amino, C₁-C₆ alkylamino, -NH(CH₂)pNH₂ wherein p is 1 to 4, -N(CH₂CH₂NH₂)₂, diazamino, amidino, and guanidino, preferably any of which is optionally substituted with one or more of amino, halo, cyano, nitro, hydroxy, -NH(CH₂)ₚNH₂ wherein p is 1 to 4, -N(CH₂CH₂NH₂)₂, amidino, guanyl, guanidino, or aminoalkoxy. Values of V that are most preferred are amino and guanidino.

Preferred values of pPL are 0 to 6; values of pPL of 0 to 4 are especially preferred, with values of pPL of 2 to 4 especially preferred.

Preferred values of q1PL and q2PL are 0 or 1. Values of q1PL and q2PL of 0 or 1 are especially preferred, with a value of 0 being especially preferred for each of q1PL and q2PL.

In preferred polymers and oligomers of Formula IV, the -(CH₂)_{pPL}-alkylene chain in PL is optionally substituted with one or more amino or hydroxy groups.

Preferred polymers of Formula IV are those in which m is 1 to about 500. Especially preferred are those polymers of Formula IV wherein m is 1 to about 100, or wherein m is 1 to about 50.

Oligomers of Formula IV that are preferred are those wherein m is 1 to about 30, or m is 1 to about 25; more preferred are those wherein m is 1 to about 20, or wherein m is 1 to about 10, or wherein m is 1 to about 5. Especially preferred are those oligomers of Formula IV wherein m is 1, 2 or 3.

Preferred oligomers of the invention include those of Formula IVa, Formula IVb, or Formula IVc:

**R¹-x-A₁-x-z-y-A₂-y-R²** (IVa)

**R¹-x-A₁-x-z-y-A₂-y-z-x-A₁-x-R²** (IVb)

**R¹-x-A₁-x-z-y-A2-y-z-x-A₁-x-z-y-A₂-y-R²** (IVc)

or an acceptable salt or solvate thereof,
wherein:
x is NR⁸, -NR⁸NR⁸-, C=O, or O; y is NR⁸ -NR⁸NR⁸- C=O, S, or O; and R⁸ is hydrogen or alkyl;
z is C=O, C=S, O=S=O, -NR⁸NR⁸-, or-C(=O)C(=O)- ;
A₁, and A₂ are independently optionally substituted arylene or optionally substituted heteroarylene, wherein A₁ and A₂ are independently optionally substituted with one or more polar (PL) group(s), one or more non-polar (NPL) group(s), or a combination of one or more polar (PL) group(s) and one or more non-polar (NPL) group(s);
R¹ is hydrogen, a polar group (PL), or a non-polar group (NPL), and R² is R¹ ;
NPL is a nonpolar group independently selected from the group consisting of -B(OR⁴)₂ and -(NR^{3'})_{qINPL}-U^{NPL}-(CH₂)_{pNPL}-(NR^{3"})_{q2NPL}-R^{4′}, wherein:
   R³, R^{3'}, and R^{3"} are independently selected from the group consisting of hydrogen, alkyl, and alkoxy;
   R⁴ and R^{4'} are independently selected from the group consisting of hydrogen, alkyl, alkenyl, alkynyl, cycloalkyl, aryl, and heteroaryl, any of which is optionally substituted with one or more alkyl or halo groups;
U^{NPL} is absent or selected from the group consisting of O, S, S(=O), S(=O)₂, NR³, -C(=O)-, -C(=O)-N=N-NR³-, -C(=O)-NR³-N=N-, -N=N-NR³-, -C(=N-N(R³)₂)-, -C(=NR³)-, -C(=O)O-, -C(=O)S-, -C(=S)-, -O-P(=O)₂O-, -R³O-, -R³S-, -S-C=N- and -C(=O)-NR³-O- , wherein groups with two chemically nonequivalent termini can adopt both possible orientations;
the -(CH₂)_{pNpL}- alkylene chain is optionally substituted with one or more amino or hydroxy groups, or is unsaturated;
pNPL is 0 to 8;
q1NPL and q2NPL are independently 0, 1 or 2;
PL is a polar group selected from the group consisting of halo, hydroxyethoxymethyl, methoxyethoxymethyl, polyoxyethylene, and -(NR^{5′})_{q1PL}-U^{PL}-(CH₂)pPL-(NR^{5"})q2PL-V, wherein:
R⁵, R^{5'}, and R^{5"} are independently selected from the group consisting of hydrogen, alkyl, and alkoxy;
U^{PL} is absent or selected from the group consisting of O, S, S(=O), S(=O)₂, NR⁵, -C(=O)-, -C(=O)-N=N-NR⁵-, -C(=O)-NR⁵-N=N-, -N=N-NR⁵-, -C(=N-N(R⁵)₂)-, -C(=NR⁵)-, -C(=O)O-, -C(=O)S-, -C(=S)-, -O-P(=O)₂O-, -R⁵O-, -R⁵S-, -S-C=N- and
-C(=O)-NR⁵-O- , wherein groups with two chemically nonequivalent termini can adopt both possible orientations;
V is selected from the group consisting of nitro, cyano, amino, hydroxy, alkoxy, alkylthio, alkylamino, dialkylamino, -NH(CH₂)pNH₂ wherein p is 1 to 4, -N(CH₂CH₂NH₂)₂, diazamino, amidino, guanidino, guanyl, semicarbazone, aryl, heterocycle and heteroaryl, preferably any of which is optionally substituted with one or more of amino, halo, cyano, nitro, hydroxy, -NH(CH₂)ₚNH₂ wherein p is 1 to 4, -N(CH₂CH₂NH₂)₂, amidino, guanidino, guanyl, aminosulfonyl, aminoalkoxy, aminoalkythio, lower acylamino, or benzyloxycarbonyl;
the -(CH₂)_{pPL}- alkylene chain is optionally substituted with one or more amino or hydroxy groups, or is unsaturated;
pPL is 0 to 8;
q1PL and q2PL are independently 0, 1 or 2; and
a pharmaceutically acceptable carrier or diluent.

Preferred values of A₁, A₂, R¹, R², R³, R^{3'}, R^{3"}, R⁴, R^{4'}, NPL, U^{NPL}, pNPL, q1NPL, q2NPL, PL, R⁵, R^{5'}, R^{5"}, V, U^{PL}, pPL, q1PL and q2PL for the oligomers of Formula IVa, Formula IVb, and Formula IVc are also the same as those listed for polymers and oligomers of Formula IV above.

Examples of oligomers of the present invention, including oligomers for use in the disclosed methods of the invention, include, but are not limited to, the following:

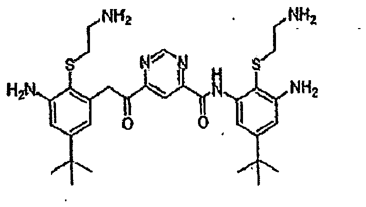

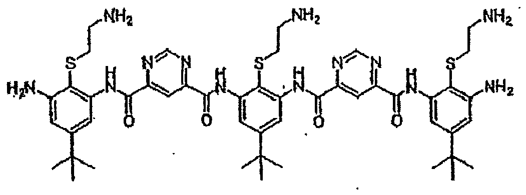

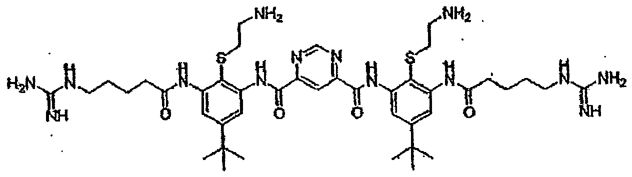

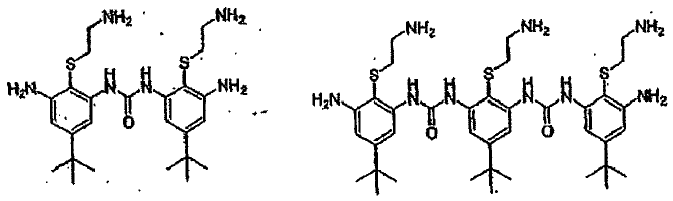

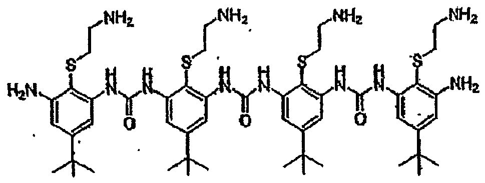

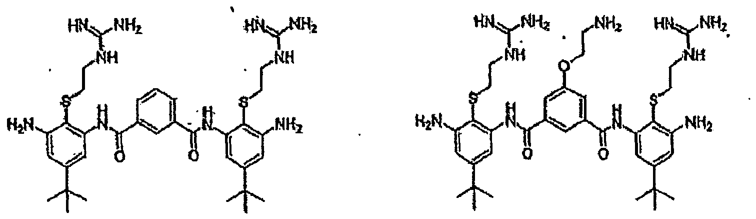

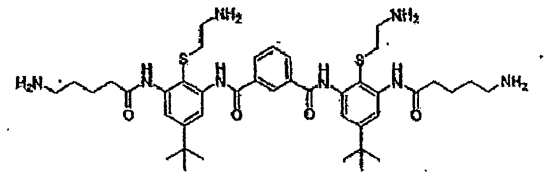

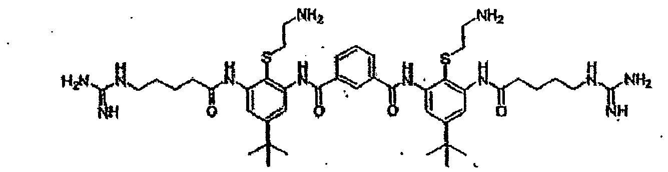

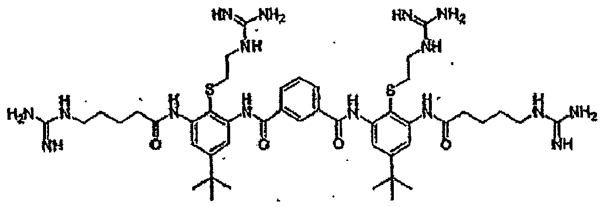

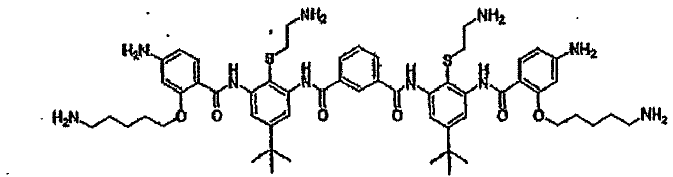

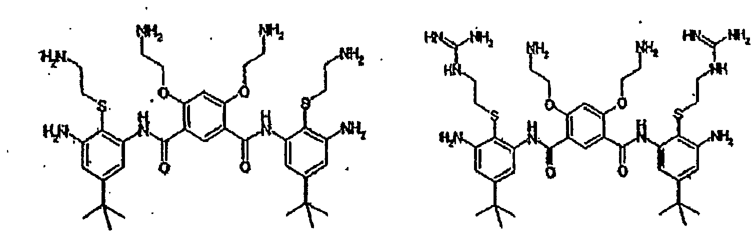

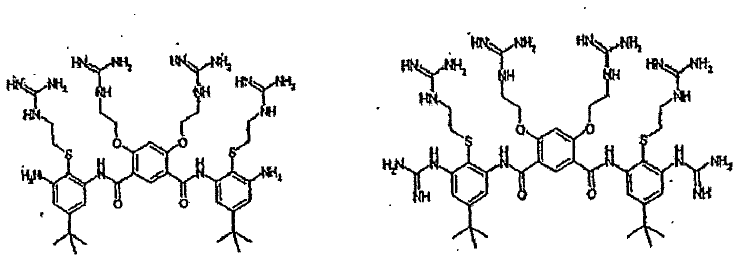

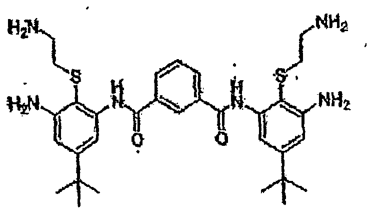

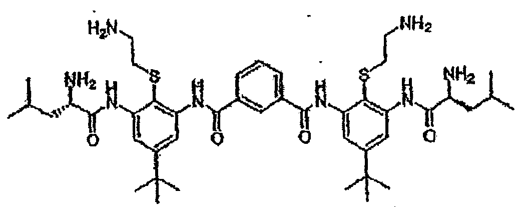

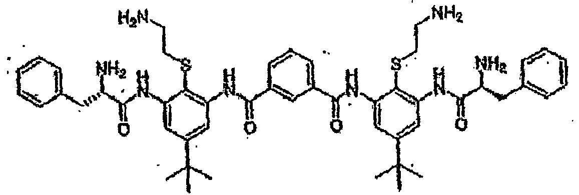

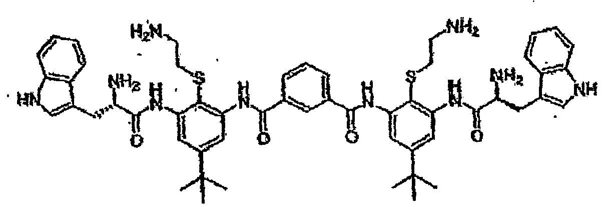

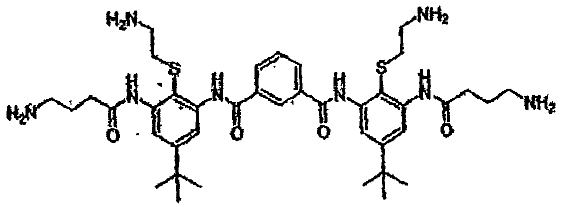

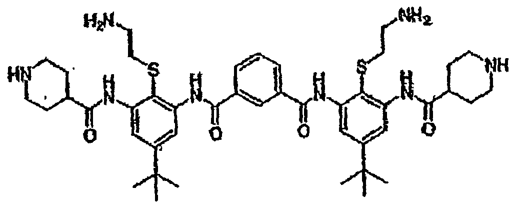

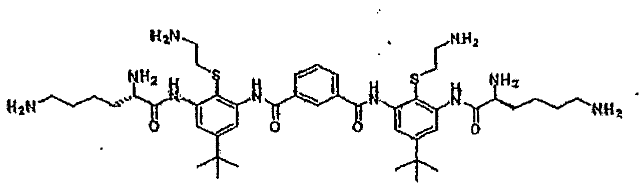

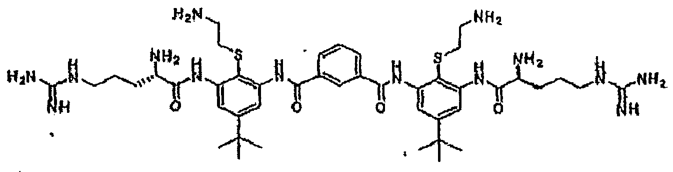

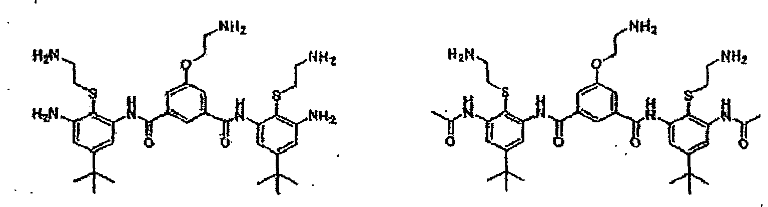

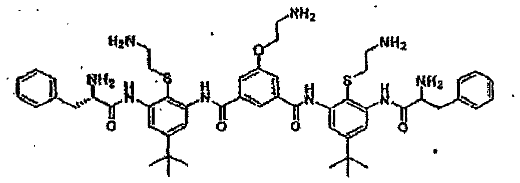

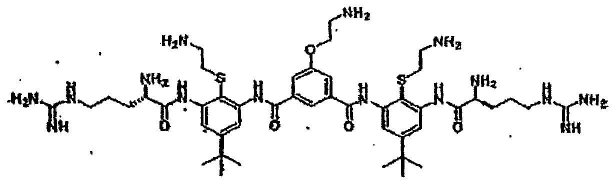

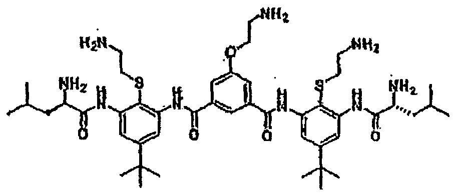

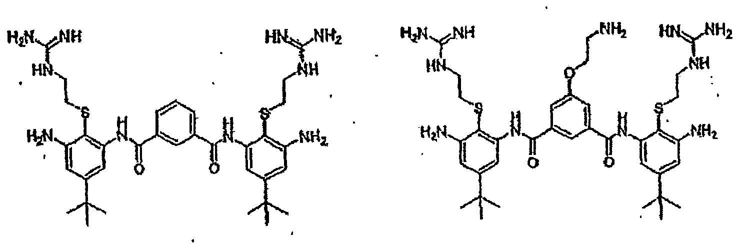

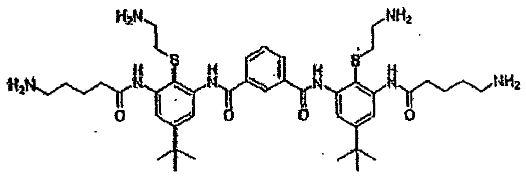

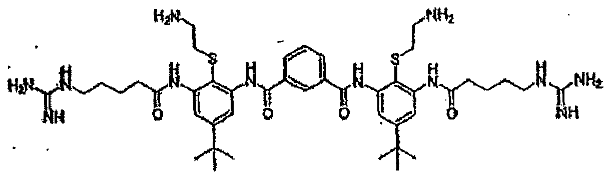

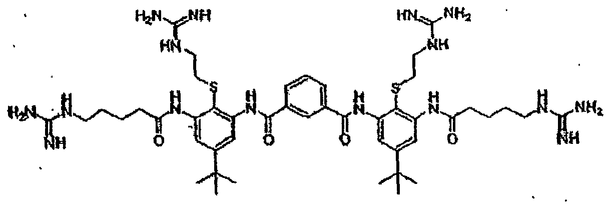

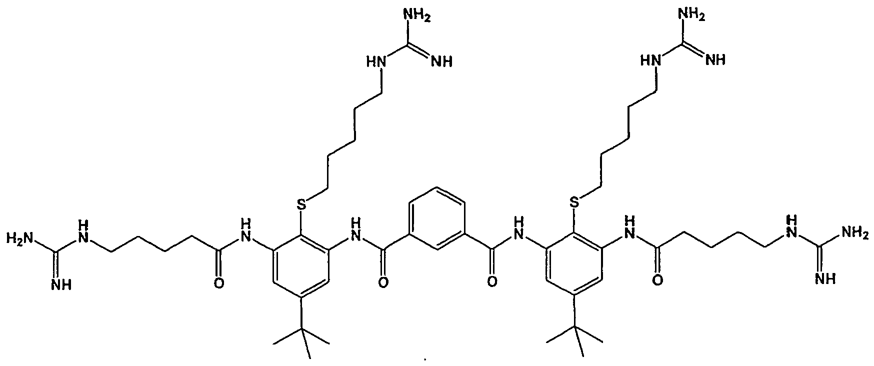

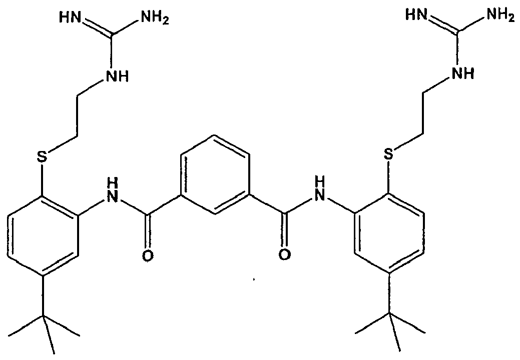

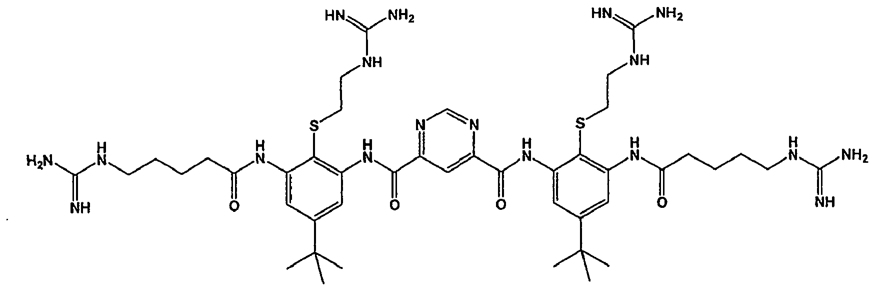

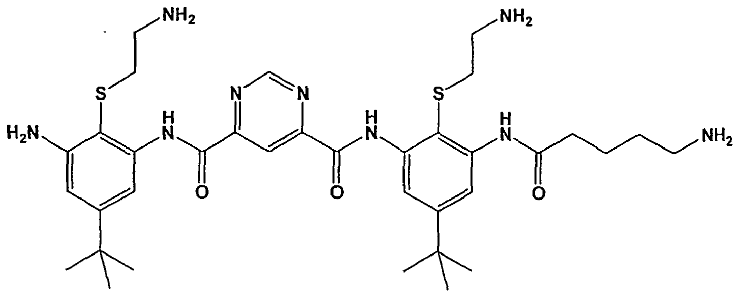

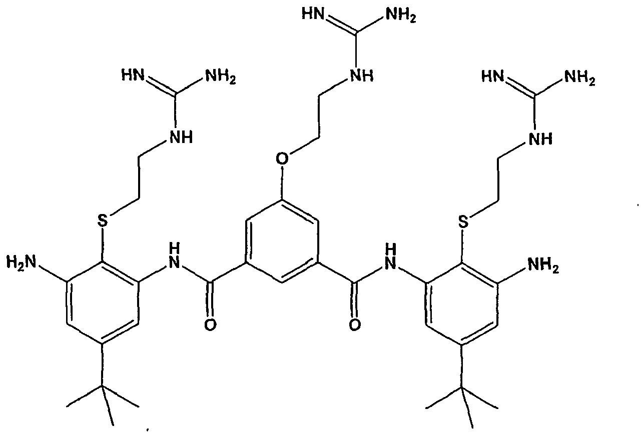

The polymers and oligomers of the present invention also include those of Formula V:

**R¹-[-A₁-s-A₂-s-]ₘ-R²** (V)

or an acceptable salt or solvate thereof,
wherein:
A₁ and A₂ are independently optionally substituted arylene or optionally substituted heteroarylene, wherein:
   (i) A₁ and A₂ are independently optionally substituted with one or more polar (PL) group(s), one or more non-polar (NPL) group(s), or a combination of one or more polar (PL) group(s) and one or more non-polar (NPL) group(s); or
   (ii) one of A₁, or A₂ is as defined above and is optionally substituted with one or more polar (PL) group(s), one or more non-polar (NPL) group(s), or a combination of one or more polar (PL) group(s) and one or more non-polar (NPL) group(s); and the other of A₁ or A₂ is the group -C≡C(CH₂)ₚC≡C-, wherein p is 0 to 8, and the -(CH₂)ₚ- alkylene chain is optionally substituted with one or more amino or hydroxyl groups;
   s is absent, or represents -CH₂-, -CH₂-CH₂-, -CH=CH- , or -C≡C- ;
R¹ is
   (i) hydrogen, a polar group (PL), or a non-polar group (NPL), and R² is -A₁-R¹, wherein A₁ is as defined above and is optionally substituted with one or more polar (PL) group(s), one or more non-polar (NPL) group(s), or a combination of one or more polar (PL) group(s) and one or more non-polar (NPL) group(s); or
   (ii) hydrogen, a polar group (PL), or a non-polar group (NPL), and R² is -A₁-s-A₂-R¹, wherein each of A₁ and A₂ is as defined above and is optionally substituted with one or more polar (PL) group(s), one or more non-polar (NPL) group(s), or a combination of one or more polar (PL) group(s) and one or more non-polar (NPL) group(s); or
   (iii) A′-s- and R² is -A₁-s-A′, wherein A′ is aryl or heteroaryl, either of which is optionally substituted with one or more polar (PL) group(s), one or more non-polar (NPL) group(s), or a combination of one or more polar (PL) group(s) and one or more non-polar (NPL) group(s); or
   (iv) A′-s- and R² is -A', wherein A' is aryl or heteroaryl, either of which is optionally substituted with one or more polar (PL) group(s), one or more non-polar (NPL) groups(s), or a combination of one or more polar (PL) group(s) and one or more non-polar (NPL) group(s); or
   (v) R¹ and R² together form a single bond;
NPL is a nonpolar group independently selected from -B(OR⁴)₂ or -(NR^{3′})_{q1NPL}-U^{NPL}-(CH₂)_{pNPL}-(NR^{3"})_{q2NPL}-R⁴, wherein:
   R³, R^{3'}, and R^{3"} are independently selected from the group consisting of hydrogen, alkyl, and alkoxy;
   R⁴ is selected from the group consisting of hydrogen, alkyl, alkenyl, alkynyl, cycloalkyl, aryl, and heteroaryl, any of which is optionally substituted with one or more alkyl or halo groups;
   U^{NPL} is absent or selected from the group consisting of O, S, S(=O), S(=O)₂, NR³, -(C=O)-, -(C=O)-N=N-NR³-, -(C=O)-NR³-N=N-, -N=N-NR³-, -C(=N-N(R³)₂)-, -C(=NR³)-, -C(=O)O-, -C(=O)S-, -C(=S)-, -O-P(=O)₂O-, -R³O-, -R³S-, -S-C=N- and -(C=O)-NR³-O-, wherein groups with two chemically nonequivalent termini can adopt both possible orientations;
   the -(CH₂)_{pNPL}- alkylene chain is optionally substituted with one or more amino or hydroxyl groups, or the alkylene chain is unsaturated;
   pNPL is 0 to 8;
   q1NPL and q2NPL are independently 0 to 2;
PL is a polar group selected from the group consisting of halo, hydroxyethoxymethyl, methoxyethoxymethyl, polyoxyethylene, and -(NR^{5'})_{q1PL}-U^{PL}-(CH₂)_{pPL}-(NR^{5"})_{q2PL}-V, wherein:
   R⁵, R^{5'}, and R^{5"} are independently selected from the group consisting of hydrogen, alkyl, and alkoxy;
   U^{PL} is absent or selected from the group consisting of O, S, S(=O), S(=O)₂, NR⁵, -(C=O)-, -(C=O)-N=N-NR⁵-, -(C=O)-NR⁵-N=N-, -N=N-NR⁵-, -C(=N-N(R⁵)₂)-_{,} -C(=NR⁵)-, -C(=O)O-, -C(=O)S-, -C(=S)-, -O-P(=O)₂O-, -R⁵O-, -R⁵S-, -S-C=N- and -(C=O)-NR⁵-O-, wherein groups with two chemically nonequivalent termini can adopt both possible orientations;
   V is selected from the group consisting of nitro, cyano, amino, hydroxyl, alkoxy, alkylthio, alkylamino, dialkylamino, -NH(CH₂)ₚNH₂, -N(CH₂CH₂NH₂)₂, diazamino, amidino, guanidino, guanyl, semicarbazone, aryl, heterocycle and heteroaryl, any of which is optionally substituted with one or more of amino, halo, cyano, nitro, hydroxyl, -NH(CH₂)ₚNH₂, -N(CH₂CH₂NH₂)₂, amidino, guanidino, guanyl, aminosulfonyl, aminoalkoxy, aminoalkythio, lower acylamino, or benzyloxycarbonyl;
   the -(CH₂)_{pPL}- alkylene chain is optionally substituted with one or more amino or hydroxyl groups, or the alkylene chain is unsaturated;
   pPL is 0 to 8;
   q1PL and q2PL are independently 0 to 2; and
m is 1 to at least about 500;
with the proviso that if A₁ and A₂ are thiophene, the polar groups cannot be 3-(propionic acid) or methoxy(diethoxy)ethyl and the nonpolar group cannot be n-dodecyl.

Polymers and oligomers of Formula V that are preferred for use in the methods of the present invention are those wherein A₁, and A₂ are independently optionally substituted *o*-*, m-,* or *p*-phenylene. Those oligomers wherein A₁ and A₂ are optionally substituted *m*-phenylene are especially preferred. Also preferred are polymers and oligomers of Formula V wherein one of A₁ or A₂ is *o-, m*-, or *p*-phenylene, and the other of A₁ or A₂ is heteroarylene. Preferred heteroarylene groups include, but are not limited to, pyridinyl, pyrimidinyl, and pyrazinyl.

Also preferred are polymers and oligomers of Formula V wherein A₁ and A₂ are independently optionally substituted arylene or optionally substituted heteroarylene, and (i) one of A₁ or A₂ is substituted with one or more polar (PL) group(s) and one or more nonpolar (NPL) group(s) and the other of A₁ or A₂ is unsubstituted; or (ii) one of A₁ or A₂ is substituted with one or more polar (PL) group(s) and the other of A₁ or A₂ is unsubstituted; or (iii) one of A₁ or A₂ is substituted with one or more polar (PL) group(s) and the other of A₁ or A₂ is substituted with one or more nonpolar (NPL) group(s). Polymers and oligomers in which either (i) one of A₁ or A₂ is substituted with one or more polar (PL) group(s) and one or more nonpolar (NPL) group(s), and the other of A₁ or A₂ is unsubstituted, or (ii) one of A₁ or A₂ is substituted with one or more polar (PL) group(s) and the other of A₁ or A₂ is unsubstituted, are especially preferred.

Polymers and oligomers of Formula V are preferred in which A₁ and A₂ are optionally substituted *m*-phenylene, wherein one of A₁ or A₂ is substituted with one or more polar (PL) group(s) and the other of A₁ or A₂ is unsubstituted. Those polymers and oligomers wherein one of A₁ or A₂ is substituted with one or two polar groups and the other of A₁ or A₂ is unsubstituted are especially preferred.

In some aspects of the invention, preferred polymers and oligomers of Formula V are those wherein s is absent.

In other aspects, polymers and oligomers of Formula V that are preferred for use in the disclosed methods are those wherein s is -CH=CH- or -C≡C-. Especially preferred are oligomers of Formula V in which s is -C≡C-.

Preferred polymers and oligomers of Formula V for are those wherein R¹ is (i) hydrogen, a polar group (PL), or a non-polar group (NPL), and R² is - A₁-R¹, wherein A₁, is as defined above and is optionally substituted with one or more polar (PL) group(s), one or more non-polar (NPL) group(s), or a combination of one or more polar (PL) group(s) and one or more non-polar (NPL) group(s); or (ii) A'-s- and R² is -A₁-s-A' wherein A' is aryl or heteroaryl, either of which is optionally substituted with one or more polar (PL) group(s), one or more non-polar (NPL) group(s), or a combination of one or more polar (PL) group(s) and one or more non-polar (NPL) group(s). More preferred are those oligomers of Formula V wherein R¹ is hydrogen or a polar group (PL), and R² is -A₁-R¹, where A₁ is optionally substituted with one or more polar (PL) group(s). Especially preferred are those oligomers wherein R¹ is a polar (PL) group, such as halo, and R² is -A₁-R¹, where A₁, is optionally substituted with one or more polar (PL) group(s).

In some aspects of the invention, preferred polymers and oligomers of Formula V are those wherein NPL is -B(OR⁴)₂, wherein R⁴ is hydrogen, alkyl, alkenyl, alkynyl, cycloalkyl, aryl, or heteroaryl, any of which is optionally substituted with one or more alkyl or halo groups.

In other aspects, preferred polymers and oligomers of Formula V include those wherein NPL is -(NR^{3'})_{q1NPL}-U^{NPL}-(CH₂)_{pNPL}-(NR^{3"})_{q2NPL}-R⁴, and R³, R^{3'}, R^{3"}, R⁴, U^{NPL}, pNPL, q1NPL and q2NPL are as defined above.

Preferred values for each of R³, R^{3'}, and R^{3"} are hydrogen, C₁-C₆ alkyl, and C₁-C₆ alkoxy. Hydrogen is an especially preferred value for R³, R^{3'}, and R^{3"}.

Preferred values of R⁴ are hydrogen, C₁-C₁₀ alkyl, C₃-C₁₈ branched alkyl, C₂-C₁₀ alkenyl, C₂-C₁₀ alkynyl, C₃-C₈ cycloalkyl, C₆-C₁₀ aryl, and heteroaryl, any of which is optionally substituted with one or more C₁-C₆ alkyl or halo groups. Values of R⁴ that are especially preferred are C₁-C₁₀ alkyl, C₃-C₁₈ branched alkyl, C₂-C₁₀ alkenyl, C₂-C₁₀ alkynyl, and C₆-C₁₀ aryl, especially phenyl.

Preferred values of U^{NPL} are O, S, S(=O), S(=O)₂, NH, -(C=O)-, -(C=O)-N=N-NH-, -(C=O)-NH-N=N-, -N=N-NH-, -C(=N-N(R³)₂)-, -C(=NR³)-, -C(=O)O-, -C(=O)S-, -C(=S)-, -O-P(=O)₂O-, -R³O-, -R³S-, -S-C=N- and -(C=O)-NR³-O- , wherein groups with two chemically nonequivalent termini can adopt both possible orientations. Especially preferred values of U^{NPL} are O, NH, -(C=O)-, -(C=O)-N=N-NH-, -(C=O)-NH-N=N-, -N=N-NH-, -C(=N-N(R³)₂)-, -C(=NR³)-, -C(=O)O-, and -R³O-. Preferred polymers and oligomers of Formula I also include those in which U^{NPL} is absent.

In some aspects of the invention, preferred polymers and oligomers of Formula V are those wherein U^{NPL} is -O-P(=O)₂O-.

Preferred values of pNPL are 0 to 6; values of pNPL of 0 to 4 are especially preferred, with values of pNPL of 0 to 2 most preferred.

Preferred values of q1NPL and q2NPL are 0 or 1. Values of q1NPL and q2NPL of 0 or 1 are especially preferred, with a value of 0 being the most preferred for each of q1NPL and q2NPL.

In preferred polymers and oligomers of Formula V, the alkylene chain in NPL is unsubstituted or unsaturated.

Especially preferred values of NPL for polymers and oligomers of Formula I include C₁-C₁₀ alkoxy, C₁-C₁₀ alkylthio, and C₁-C₁₀ alkylamino.

In some aspects of the invention, preferred polymer and oligomers of Formula V for use in the disclosed methods include those wherein PL is halo. Especially preferred values of PL are bromo and iodo.

In other aspects of the invention, preferred polymers and oligomers of Formula V are those wherein PL is -(NR^{5'})_{q1PL}-U^{PL}-(CH₂)_{pPL}-(NR^{5"})_{q2PL} -V, and R⁵, R^{5'}, R^{5"}, V, U^{PL}, pPL, q1PL and q2PL are as defined above.

Preferred values for R⁵, R^{5'}, and R^{5"} are hydrogen, C₁-C₆ alkyl, and C₁-C₆ alkoxy. Hydrogen is an especially preferred value for each of R⁵, R^{5'}, and R^{5"}.

Preferred values of U^{PL} are O, S, S(=O), S(=O)₂, NH, -(C=O)-, -(C=O)-N=N-NH-, -(C=O)-NH-N=N-, -N=N-NH-, -C(=N-N(R⁵)₂)-, -C(=NR⁵)-, -C(=O)O-, -C(=O)S-, -C(=S)-, -O-P(=O)₂O-, -R⁵O-, -R⁵S-, -S-C=N- and -(C=O)-NR⁵-O- , wherein groups with two chemically nonequivalent termini can adopt both possible orientations. Especially preferred values of U^{PL} are O, NH, -(C=O)-, -(C=O)-N=N-NH-, -(C=O)-NH-N=N-, -N=N-NH-, -C(=N-N(R⁵)₂)-, -C(=NR⁵)-, -C(=O)O-, and -R⁵O-. Preferred polymers and oligomers of Formula I are also those in which U^{PL} is absent.

In some aspects of the invention, preferred polymers and oligomers of Formula V are those wherein U^{PL} is -O-P(=O)₂O-.

Preferred values of V are nitro, cyano, amino, hydroxyl, C₁-C₆ alkoxy, C₁-C₆ alkylthio, C₁-C₆ alkylamino, C₁-C₆ dialkylamino, -NH(CH₂)pNH₂, -N(CH₂CH₂NH₂)₂, diazamino, amidino, guanidino, guanyl semicarbazone, C₆-C₁₀ aryl, heterocycle, and heteroaryl, any of which is optionally substituted with one or more of amino, halo, cyano, nitro, hydroxyl, -NH(CH₂)ₚNH₂. -N(CH₂CH₂NH₂)₂, amidino, guanidino, guanyl, aminosulfonyl, aminoalkoxy, lower acylamino, or benzyloxycarbonyl.

Suitable heteroaryl groups include 1,2,3-triazole, 1,2,4-triazole, 5-amino-1,2,4-triazole, imidazole, oxazole, isoxazole, 1,2,3-oxadiazole, 1,2,4-oxadizaole, 3-amino-1,2,4-oxadizaole, 1,2,5-oxadiazole, 1,3,4-oxadiazole, pyridine, and 2-aminopyridine.

Especially preferred values of V are amino, C₁-C₆ alkylamino, C₁-C₆ dialkylamino, -NH(CH₂)ₚNH₂, -N(CH₂CH₂NH₂)₂, diazamino, amidino, guanidino, guanyl, and semicarbazone, any of which is optionally substituted with one or more of amino, halo, cyano, nitro, hydroxyl, -NH(CH₂)ₚNH₂, -N(CH₂CH₂NH₂)₂, amidino, guanidino, guanyl, aminosulfonyl, aminoalkoxy, lower acylamino, or benzyloxycarbonyl.

Even more preferred values of V are amino, C₁-C₆ alkylamino, -NH(CH₂)ₚNH₂, -N(CH₂CH₂NH₂)₂, diazamino, amidino, and guanidino, any of which is optionally substituted with one or more of amino, halo, cyano, nitro, hydroxyl, -NH(CH₂)ₚNH₂, -N(CH₂CH₂NH₂)₂, amidino, guanyl, guanidine, or aminoalkoxy.

Preferred values of pPL are 0 to 6; values of pPL of 0 to 4 are especially preferred, with values of pPL of 0 to 2 especially preferred.

Preferred values of q1PL and q2PL are 0 or 1. Values of q1PL and q2PL of 0 or 1 are especially preferred, with a value of 0 being especially preferred for each of q1PL and q2PL.

In preferred polymers and oligomers of Formula V, the alkylene chain in PL is optionally substituted with one or more amino or hydroxy groups.

Preferred polymers of Formula V are those in which m is I to about 500. Especially preferred are those polymers of Formula V wherein m is 1 to about 100, or wherein m is 1 to about 50.

Oligomers of Formula V that are preferred are those wherein m is 1 to about 25; more preferred are those in which m is 1 to about 20, or in which m is 1 to about 10, or in which m is 1 to about 5. Especially preferred are those oligomers of Formula V wherein m is 1, 2 or 3.

In some aspects of the invention, preferred polymers and oligomers of Formula V are those wherein:
A₁ and A₂ are independently optionally substituted *o-, m*-, or *p*-phenylene, wherein
   (i) one of A₁ or A₂ is substituted with one or more polar (PL) group(s) and one or more nonpolar (NPL) group(s), and the other of A₁ or A₂ is unsubstituted; or
   (ii) one of A₁ or A₂ is substituted with one or more polar (PL) group(s) and the other of A₁ or A₂ is unsubstituted; or
   (iii) one of A₁ or A₂ is substituted with one or more polar (PL) group(s) and the other of A₁ or A₂ is substituted with one or more nonpolar (NPL) group(s); s is -C≡C- ;
R¹ is
   (i) hydrogen, a polar group (PL), or a non-polar group (NPL), and R² is -A₁-R¹, wherein A₁ is as defined above and is optionally substituted with one or more polar (PL) group(s), one or more non-polar (NPL) group(s), or a combination of one or more polar (PL) group(s) and one or more non-polar (NPL) group(s); or
   (ii) A'-s- and R² is -A₁-s-A', wherein A' is aryl or heteroaryl, either of which is optionally substituted with one or more polar (PL) group(s), one or more non-polar (NPL) group(s), or a combination of one or more polar (PL) group(s) and one or more non-polar (NPL) group(s);
NPL is -(NR^{3'})_{q1NPL}-U^{NPL}-(CH₂)_{pNPL}-(NR^{3"})_{q2NPL}-R⁴, wherein
   R³, R^{3'}, and R^{3"} are independently selected from the group consisting of hydrogen, C₁-C₆ alkyl, and C₁-C₆ alkoxy;
   R⁴ is selected from the group consisting of hydrogen, C₁-C₁₀ alkyl, C₃-C₁₈ branched alkyl, C₂-C₁₀ alkenyl, C₂-C₁₀ alkynyl, C₃-C₈ cycloalkyl, C₆-C₁₀ aryl, and heteroaryl, any of which is optionally substituted with one or more C₁-C₆ alkyl or halo groups;
   U^{NPL} is absent or selected from the group consisting of O, S, S(=O), S(=O)₂, NH, -(C=O)-, -(C=O)-N=N-NH-, -(C=O)-NH-N=N-, -N=N-NH-, -C(=N-N(R³)₂)-, -C(=NR³)-, -C(=O)O-, -C(=O)S-, -C(=S)-, -O-P(=O)₂O-, -R³-O-, -R³-S-, -S-C=N- and -(C=O)-NR³-O-, wherein groups with two chemically nonequivalent termini can adopt both possible orientations;
   the alkylene chain -(CH₂)_{pNPL}- is optionally substituted with one or more amino or hydroxyl groups;
   pNPL is 0 to 6;
   q1NPL and q2NPL are independently 0 or 1;
PL is halo or ―(NR^{5'})_{q1PL}-U^{PL}-(CH2)_{pPL}-(NR^{5"})_{q2PL}-V, wherein;
   R⁵, R^{5'}, and R^{5"} are independently selected from the group consisting of hydrogen, C₁-C₆ alkyl, and C₁-C₆ alkoxy;
   U^{PL} is absent or selected from the group consisting of O, S, S(=O), S(=O)₂, NH, -(C=O)-, -(C=O)-N=N-NH-, -(C=O)-NH-N=N-, -N=N-NH-, -C(=N-N(R⁵)₂)-, -C(=NR⁵)-, -C(=O)O-, -C(=O)S-, -C(=S)-, -O-P(=O)₂O-, -R⁵O-, -R⁵S-, -S-C=N- and -(C=O) -NR⁵-O-, wherein groups with two chemically nonequivalent termini can adopt both possible orientations;
   V is selected from the group consisting of nitro, cyano, amino, hydroxyl, C₁-C₆ alkoxy, C₁-C₆ alkylthio, C₁-C₆ alkylamino, C₁-C₆ dialkylamino, -NH(CH₂)pNH₂, -N(CH₂CH₂NH₂)₂, diazamino, amidino, guanidino, guanyl, semicarbazone, C₆-C₁₀ aryl, heterocycle, and heteroaryl, any of which is optionally substituted with one or more of amino, halo, cyano, nitro, hydroxyl, -NH(CH₂)_{P}NH₂, -N(CH₂CH₂NH₂)₂, amidino, guanidino, guanyl, aminosulfonyl, aminoalkoxy, lower acylamino, or benzyloxycarbonyl;
   the alkylene chain -(CH₂)_{pPL}-is optionally substituted with one or more amino or hydroxyl groups;
   pPL is 0 to 6;
   q1PL and q2PL are independently 0 or 1; and
m is 1 to about 5.

Oligomers of the present invention also include oligomers of Formula Va,

**R¹-A₁-s-A₂-s-A₁-R²** (Va)

or an acceptable salt or solvate thereof,
wherein:
A₁ and A₂ are independently optionally substituted arylene or optionally substituted heteroarylene, wherein:
   (i) A₁ and A₂ are independently optionally substituted with one or more polar (PL) group(s), one or more non-polar (NPL) group(s), or a combination of one or more polar (PL) group(s) and one or more non-polar (NPL) group(s); or
   (ii) one of A₁ or A₂ is as defmed above and is optionally substituted with one or more polar (PL) group(s), one or more non-polar (NPL) group(s), or a combination of one or more polar (PL) group(s) and one or more non-polar (NPL) group(s); and the other of A₁ or A₂ is the group -C≡C(CH₂)ₚC≡C-, wherein p is 0 to 8, and the -(CH₂)ₚ- alkylene chain is optionally substituted with one or more amino or hydroxyl groups;
      s is absent, or is -CH=CH- or -C≡C- ;
R¹ is hydrogen, a polar group (PL), a non-polar group (NPL), or -s-A', wherein A' is aryl or heteroaryl, either of which is optionally substituted with one or more polar (PL) group(s), one or more non-polar (NPL) group(s), or a combination of one or more polar (PL) group(s) and one or more non-polar (NPL) group(s);
R² is R¹;
NPL is a nonpolar group independently selected from -B(OR⁴)₂ or -(NR^{3'})_{q1NPL}-U^{NPL}-(CH₂)_{pNPL}-(NR^{3"})_{q2NPL} -R⁴, wherein
   R³, R^{3'}, and R^{3"} are independently selected from the group consisting of hydrogen, alkyl, and alkoxy;
   R⁴ is selected from the group consisting of hydrogen, alkyl, alkenyl, alkynyl, cycloalkyl, aryl, and heteroaryl, any of which is optionally substituted with one or more alkyl or halo groups;
   U^{NPL} is absent or selected from the group consisting of O, S, S(=O), S(=O)_{2,} NR³, -(C=O)-, -(C=O)-N=N-NR³-, -(C=O)-NR³-N=N-, -N=N-NR³-, -C(=N-N(R³)₂)-, -C(=_{NR}³)-, -C(=O)O-, -C(=O)S-, -C(=S)-, -O-P(=O)₂O-, -R³-O-, -R³-S-, -S-C=N- and -(C=O)-NR³-O-, wherein groups with two chemically nonequivalent termini can adopt both possible orientations;
   the alkylene chain -(CH₂)_{pNPL}- is optionally substituted with one or more amino or hydroxyl groups, or the alkylene chain is unsaturated;
   pNPL is 0 to 8;
   q1NPL and q2NPL are independently 0 to 2;
PL is a polar group selected from the group consisting of halo, hydroxyethoxymethyl, methoxyethoxymethyl, polyoxyethylene, and -(NR^{5'})_{q1PL}-U^{PL}-(CH₂)_{pPL}-(NR^{5"})_{q2PL}-V, wherein;
   R⁵, R^{5'}, and R^{5"} are independently selected from the group consisting of hydrogen, alkyl, and alkoxy;
   U^{PL} is absent or selected from the group consisting of O, S, S(=O), S(=O)₂, NR⁵, -(C=O)-, -(C=O)-N=N-NR⁵-, -(C=O)-NR⁵-N=N-, -N=N-NR⁵-, -C(=N-N(R⁵)₂)-, -C(=NR⁵)-, -C(=O)O-, -C(=O)S-, -C(=S)-, -O-P(=O)₂O-, -R⁵O-, -R⁵S-, -S-C=N- and -(C=O)-NR⁵-O- , wherein groups with two chemically nonequivalent termini can adopt both possible orientations;
   V is selected from the group consisting of nitro, cyano, amino, hydroxyl, alkoxy, alkylthio, alkylamino, dialkylamino, -NH(CH₂)pNH₂, -N(CH₂CH₂NH₂)₂, diazamino, amidino, guanidino, guanyl, semicarbazone, aryl, heterocycle and heteroaryl, any of which is optionally substituted with one or more of amino, halo, cyano, nitro, hydroxyl, -NH(CH₂)ₚNH₂, -N(CH₂CH₂NH₂)₂, amidino, guanidino, guanyl, aminosulfonyl, aminoalkoxy, aminoalkythio, lower acylamino, or benzyloxycarbonyl;
the alkylene chain -(CH₂)_{pPL}- is optionally substituted with one or more amino or hydroxyl groups, or the alkylene chain is unsaturated;
pPL is 0 to 8; and
q1PL and q2PL are independently 0 to 2;
with the proviso that if A₁ and A₂ are thiophene, the polar groups cannot be 3-(propionic acid) or methoxy(diethoxy)ethyl and the nonpolar group cannot be n-dodecyl.

A preferred group of oligomers falling within the scope of the present invention include oligomers of Formula Va wherein A₁ and A₂ are independently optionally substituted *o-* , *m*- or *p*-phenylene. Oligomers of Formula Va wherein A₁ and A₂ are optionally substituted *m*-phenylene are especially preferred. Also preferred are oligomers of Formula Va wherein one of A₁ or A₂ is *o-, m-,* or *p*-phenylene, and the other of A₁ or A₂ is heteroarylene. Preferred heteroarylene groups include, but are not limited to, pyridinyl, pyrimidinyl, and pyrazinyl.

Especially preferred oligomers of Formula Va are those wherein A₁ and A₂ are optionally substituted *m*-phenylene.

Preferred oligomers of Formula Va include those wherein one of A₁ or A₂ is substituted with one or more polar (PL) group(s) and one or more nonpolar (NPL) group(s) and the other of A₁ or A₂ is unsubstituted. Preferred oligomers also include those wherein one of A₁ or A₂ is substituted with one or more polar (PL) group(s) and the other of A or A₂ is unsubstituted.

Preferred oligomers include those wherein s is -C≡C-.

A preferred group of oligomers having Formula Va include oligomers wherein R¹ is hydrogen, a polar group (PL), or a non-polar group (NPL); and R² is R¹.

Especially preferred are oligomers of Formula Va wherein R¹ is selected from the group consisting of hydrogen, halo, nitro, cyano, C₁-C₆ alkoxy, C₁-C₆ alkoxycarbonyl, and benzyloxycarbonyl. Oligomers of Formula Va wherein R¹ and R² are halo are especially preferred.

Preferred oligomers that fall within the scope of Formula Va include those wherein NPL is ―(NR^{3'})_{q1NPL}-U-(CH₂)_{pNPL}-(NR^{3"})_{q2NPL}-R⁴, and R³, R^{3'}, R^{3"}, R⁴, U^{NPL}, pNPL, q1NPL and q2NPL are as defined above. Those oligomers wherein q1NPL and q2NPL are independently 0 or 1 are preferred.

Preferred R³, R^{3'}, and R^{3"} groups include hydrogen and C₁-C₄ alkyl. Especially preferred are those oligomers of Formula Va wherein R³, R^{3'}, and R^{3"} are each hydrogen.

Preferred R⁴ groups include hydrogen, C₁-C₁₀ alkyl, C₃-C₁₈ branched alkyl, C₂-C₁₀ alkenyl, C₂-C₁₀ alkynyl, C₃-C₈ cycloalkyl, C₆-C₁₀ aryl, and heteroaryl, any of which is optionally substituted with one or more C₁-C₆ alkyl or halo groups. Especially preferred are oligomers wherein R⁴ is C₁-C₁₀ alkyl, C₃-C₁₈ branched alkyl, C₂-C₁₀ alkenyl, C₂-C₁₀ alkynyl, or C₆-C₁₀ aryl, especially phenyl. Oligomers wherein R⁴ is hydrogen, C₁-C₁₀ alkyl, and C₃-C₁₈ branched alkyl, any of which is optionally substituted with one or more C₁-C₄ alkyl or halo groups, are especially preferred.

Preferred oligomers of Formula Va are those wherein U^{NPL} is O, S, S(=O), S(=O)₂, NH, -(C=O)-, -(C=O)-N=N-NH-, -(C=O)-NH-N=N-, -N=N-NH-, -C(=N-N(R³)₂)-, -C(=NR³)-, -C(=O)O-, -C(=O)S-, -C(=S)-, -O-P(=O)₂O-, -R³O-, -R³S-, -S-C=N- or -(C=O)-NR³-O-, wherein groups with two chemically nonequivalent termini can adopt both possible orientations. Oligomers of Formula Va wherein U^{NPL} is O, S, or -(C=O)- are especially preferred. Oligomers of Formula Va wherein U^{NPL} is absent are also preferred.

Preferred oligomers are those wherein NPL is *n*-pentoxy, *n*-butoxy, *sec-*butoxy, *tert*-butoxy, propyloxy, ethyloxy, methoxy, or phenoxy.

Preferred oligomers of Formula Va are those wherein one or more PL are halo, especially bromo or iodo.

Preferred oligomers that fall within the scope of Formula Va also include those wherein PL is ―(NR^{5'})_{q1PL}-U^{PL}-(CH₂)_{pPL}-(NR^{5"})_{q2PL} -V, and R⁵, R^{5'}, R^{5"}, V, U^{PL}, pPL, and q1PL and q2PL are as defined above.

Preferred values for R⁵, R^{5'}, and R^{5"} are hydrogen, C₁-C₆ alkyl, and C₁-C₆ alkoxy. Hydrogen is an especially preferred value for each of R⁵, R^{5'}, and R^{5"}.

Preferred values of U^{PL} are O, S, S(=O), S(=O)₂, NH, -(C=O)-, -(C=O)-N=N-NH-, -(C=O)-NH-N=N-, -N=N-NH-, -C(=N-N(R⁵)₂)-, -C(=NR⁵)-, -C(=O)O-, -C(=O)S-, -C(=S)-, -R⁵O-, -R⁵S-, -S-C=N- and -(C=O)-NR⁵-O- , wherein groups with two chemically nonequivalent termini can adopt both possible orientations. Also preferred are oligomers of Formula Va wherein U^{PL} is absent. Oligomers of Formula Va wherein U^{PL} is O, S, -(C=O)-, -C(=O)O-, -C(=O)S- , or absent, are preferred. Those oligomers wherein U^{PL} is -(C=O)- or absent are especially preferred.

In some aspects of the invention, preferred polymers and oligomers of Formula Va are those wherein U^{PL} is -O-P(=O)₂O-.

Preferred oligomers of Formula Va are those wherein q1PL and q2PL are independently 0 or 1.

Preferred oligomers of Formula Va are those wherein V is nitro, cyano, amino, hydroxyl, C₁-C₆ alkoxy, C₁-C₆ alkylthio, C₁-C₆ alkylamino, C₁-C₆ dialkylamino, -NH(CH₂)ₚNH₂, -N(CH₂CH₂NH₂)₂, diazamino, amidino, guanidino, guanyl, semicarbazone, heterocycle, or heteroaryl, any of which is optionally substituted with one or more of amino, halo, cyano, nitro, hydroxyl, -NH(CH₂)ₚNH₂, -N(CH₂CH₂NH₂)₂, amidino, guanyl, guanidine, or aminoalkoxy. Especially preferred values of V include amino, C₁-C₆ alkylamino, C₁-C₆ dialkylamino, -NH(CH₂)ₚNH₂, -N(CH₂CH₂NH₂)₂, diazamino, amidino, guanidino, or guanyl, any of which is optionally substituted with one or more of amino, halo, -NH(CH₂)ₚNH₂, -N(CH₂CH₂NH₂)₂, amidino, guanyl, guanidine, or aminoalkoxy.

Suitable heteroaryl groups include 1,2,3-triazole, 1,2,4-triazole, 5-amino-1,2,4-triazole, imidazole, oxazole, isoxazole, 1,2,3-oxadiazole, 1,2,4-oxadizaole, 3-amino-1,2,4-oxadizaole, 1,2,5-oxadiazole, 1,3,4-oxadiazole, pyridine, or 2-aminopyridine.

Especially preferred oligomers include those wherein PL is halo, guanidinomethyl, guanidinoethyl, guanidinopropyl, aminomethyl, aminoethyl, aminopropyl, aminoethylaminocarbonyl, or aminomethylaminocarbonyl.

Preferred oligomers of Formula Va are those wherein pPL is 0 to 4. Especially preferred are those oligomers wherein pPL is 0 to 2.

Exemplary structures of oligomers of Formula Va within the scope of the invention include the following: 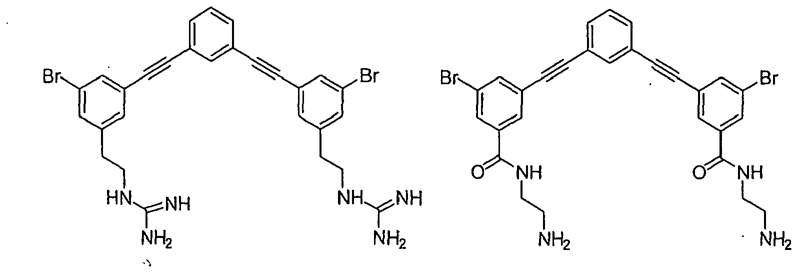 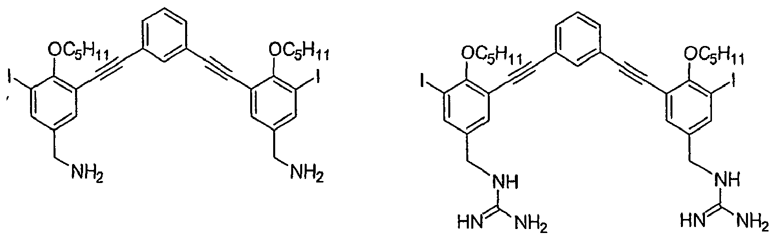 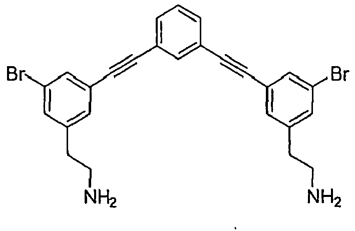 or physiologically acceptable salts thereof.

In some aspects of the invention, the polymers and oligomers of the present invention (e.g., the polymers and/or oligomers of Formulae I, II, IIa, IV, IVa, IVb, IVc, V, or Va) are derivatives referred to as prodrugs. The expression "prodrug" denotes a derivative of a known direct acting drug, which derivative has enhanced delivery characteristics and therapeutic value as compared to the drug, and is transformed into the active drug by an enzymatic or chemical process.

When any variable occurs more than one time in any constituent or in any of the polymers or oligomers recited for any of the general Formulae above (for example, in Formula I, Formula II, Formula IIa, Formula IV, Formula IVa, Formula IVb, Formula IVc, Formula V, or Formula Va), its definition on each occurrence is independent of its definition at every other occurrence. Also, combinations of substituents and/or variables are permissible only if such combinations result in stable compounds.

It is understood that the present invention encompasses the use of stereoisomers, diastereomers and optical isomers of the polymers of the present invention, as well as mixtures thereof, for use in the methods disclosed herein. Additionally, it is understood that stereoisomers, diastereomers and optical isomers of the polymers and oligomers of the present invention, and mixtures thereof, are within the scope of the invention. By way of nonlimiting example, the mixture can be a racemate or the mixture may comprise unequal proportions of one particular stereoisomer over the other. Thus, in some aspects of the invention, the polymers and oligomers of the invention are provided as mixtures that are racemates. Additionally, the polymers and oligomers of the invention can be provided as a substantially pure stereoisomers, diastereomers and optical isomers. Thus, in some aspects of the invention, the polymers and oligomers are provided as substantially pure stereoisomers, diastereomers, or optical isomers.

In another aspect of the invention, the polymers and oligomers of the invention are provided in the form of an acceptable salt (i.e., a pharmaceutically acceptable salt) for treating microbial infections, killing or inhibiting the growth of a microorganism, and providing an antidote to low molecular weight heparin overdose in an animal. Polymer or oligomer salts can be provided for pharmaceutical use, or as an intermediate in preparing the pharmaceutically desired form of the polymer or oligomer. One polymer or oligomer salt that is considered to be acceptable is the hydrochloride acid addition salt. Hydrochloride acid addition salts are often acceptable salts when the pharmaceutically active agent has an amine group that can be protonated. Since a polymer or oligomer of the invention may be polyionic, such as a polyamine, the acceptable polymer or oligomer salt can be provided in the form of a poly(amine hydrochloride).

Unless otherwise defined, the terms below have the following meanings.

The term "alkyl" as used herein by itself or as part of another group refers to both straight and branched chain radicals from 1 to 12 carbons, such as methyl, ethyl, propyl, isopropyl, butyl, t-butyl, isobutyl, pentyl, hexyl, isohexyl, heptyl, 4,4-dimethylpentyl, octyl, 2,2,4-trimethylpentyl, nonyl, decyl, undecyl, dodecyl.

The term "alkenyl," as used herein refers to a straight or branched chain radical of 2-20 carbon atoms, unless the chain length is limited thereto, including, but not limited to, ethenyl, 1-propenyl, 2-propenyl, 2-methyl-1-propenyl, 1-butenyl, 2-butenyl, and the like. Preferably, the alkenyl chain is 2 to 10 carbon atoms in length, more preferably, 2 to 8 carbon atoms in length most preferably from 2 to 4 carbon atoms in length.

The term "alkynyl," as used herein refers to a straight or branched chain radical of 2-20 carbon atoms, unless the chain length is limited thereto, wherein there is at least one triple bond between two of the carbon atoms in the chain, including, but not limited to, acetylene, 1-propylene, 2-propylene, and the like. Preferably, the alkynyl chain is 2 to 10 carbon atoms in length, more preferably, 2 to 8 carbon atoms in length, most preferably from 2 to 4 carbon atoms in length.

The term "alkylene" as used herein refers to an alkyl linking group, *i.e.,* an alkyl group that links one group to another group in a molecule.

The term "alkoxy" as used herein refers to mean a straight or branched chain radical of 1 to 20 carbon atoms, unless the chain length is limited thereto, bonded to an oxygen atom, including, but not limited to, methoxy, ethoxy, n-propoxy, isopropoxy, and the like. Preferably the alkoxy chain is 1 to 10 carbon atoms in length, more preferably 1 to 8 carbon atoms in length, and even more preferred 1 to 6 carbon atoms in length.

The term "aryl" as used herein by itself or as part of another group refers to monocyclic or bicyclic aromatic groups containing from 6 to 12 carbons in the ring portion, preferably 6-10 carbons in the ring portion, such as the carbocyclic groups phenyl, naphthyl or tetrahydronaphthyl. The term "aryl" can represent carbocyclic aryl groups, such as phenyl, naphthyl or tetrahydronaphthyl, as well as heterocyclic aryl ("heteroaryl") groups, such as pyridyl, pyrimidinyl, pyridazinyl, furyl, and pyranyl.

The term "arylene" as used herein by itself or as part of another group refers to an aryl linking group, *i.e.,* an aryl group that links one group to another group in a molecule.

The term "cycloalkyl" as used herein by itself or as part of another group refers to cycloalkyl groups containing 3 to 9 carbon atoms, more preferably, 3 to 8 carbon atoms. Typical examples are cyclopropyl, cyclobutyl, cyclopentyl, cyclohexyl, cycloheptyl, cyclooctyl and cyclononyl.

The term "halogen" or "halo" as used herein by itself or as part of another group refers to chlorine, bromine, fluorine or iodine.

The term "hydoxy" or "hydroxyl" as used herein by itself or as part of another group refers to an -OH group.

The term "heteroaryl" as used herein refers to groups having 5 to 14 ring atoms; 6, 10 or 14 π-electrons shared in a cyclic array; and containing carbon atoms and 1, 2 or 3 oxygen, nitrogen or sulfur heteroatoms. Examples of heteroaryl groups include thienyl, imadizolyl, oxadiazolyl, isoxazolyl, triazolyl, pyridyl, pyrimidinyl, pyridazinyl, furyl, pyranyl, thianthrenyl, pyrazolyl, pyrazinyl, indolizinyl, isoindolyl, isobenzofuranyl, benzoxazolyl, xanthenyl, 2H-pyrrolyl, pyrrolyl, 3H-indolyl, indolyl, indazolyl, purinyl, 4H-quinolizinyl, isoquinolyl, quinolyl, phthalazinyl, naphthyridinyl, quinazolinyl, phenanthridinyl, acridinyl, perimidinyl, phenanthrolinyl, phenazinyl, isothiazolyl, phenothiazinyl, isoxazolyl, furazanyl, and phenoxazinyl groups. Especially preferred heteroaryl groups include 1,2,3-triazole, 1,2,4-triazole, 5-amino-1,2,4-triazole, imidazole, oxazole, isoxazole, 1,2,3-oxadiazole, 1,2,4-oxadiazole, 3-amino-1,2,4-oxadiazole, 1,2,5-oxadiazole, 1,3,4-oxadiazole, pyridine, and 2-aminopyridine.

The term "heteroarylene" as used herein by itself or as part of another group refers to a heteroaryl linking group, *i.e.*, a heteroaryl group that links one group to another group in a molecule.

The term "heterocycle" or "heterocyclic ring", as used herein except where noted, represents a stable 5- to 7-membered mono- or bicyclic or stable 7- to 10-membered bicyclic heterocyclic ring system any ring of which may be saturated or unsaturated, and which consists of carbon atoms and from one to three heteroatoms selected from the group consisting of N, O and S, and wherein the nitrogen and sulfur heteroatoms may optionally be oxidized, and the nitrogen heteroatom may optionally be quaternized, and including any bicyclic group in which any of the above-defined heterocyclic rings is fused to a benzene ring. Especially useful are rings containing one oxygen or sulfur, one to three nitrogen atoms, or one oxygen or sulfur combined with one or two nitrogen atoms. The heterocyclic ring may be attached at any heteroatom or carbon atom which results in the creation of a stable structure. Examples of such heterocyclic groups include piperidinyl, piperazinyl, 2-oxopiperazinyl, 2-oxopiperidinyl, 2-oxopyrrolodinyl, 2-oxoazepinyl, azepinyl, pyrrolyl, 4-piperidonyl, pyrrolidinyl, pyrazolyl, pyrazolidinyl, imidazolyl, imidazolinyl, imidazolidinyl, pyridyl, pyrazinyl, pyrimidinyl, pyridazinyl, oxazolyl, oxazolidinyl, isoxazolyl, isoxazolidinyl, morpholinyl, thiazolyl, thiazolidinyl, isothiazolyl, quinuclidinyl, isothiazolidinyl, indolyl, quinolinyl, isoquinolinyl, benzimidazolyl, thiadiazoyl, benzopyranyl, benzothiazolyl, benzoxazolyl, furyl, tetrahydrofuryl, tetrahydropyranyl, thienyl, benzothienyl, thiamorpholinyl, thiamorpholinyl sulfoxide, thiamorpholinyl sulfone, and oxadiazolyl. Morpholino is the same as morpholinyl.

The term "alkylamino" as used herein by itself or as part of another group refers to an amino group which is substituted with one alkyl group having from 1 to 6 carbon atoms. The term "dialkylamino" as used herein by itself or as part of an other group refers to an amino group which is substituted with two alkyl groups, each having from 1 to 6 carbon atoms.

The term "alkylthio" as used herein by itself or as part of an other group refers to a thio group which is substituted with one alkyl group having from 1 to 6 carbon atoms.

The term "lower acylamino" as used herein by itself or as part of an other group refers to an amino group substituted with a C₁-C₆ alkylcarbonyl group.

The term "chemically nonequivalent termini" as used herein refers to a functional group such as an ester, amide, sufonamide, or N-hydroxyoxime that, when reversing the orientation of the functional group (e.g., -(C=O)O-) produces different chemical entities (e.g., -R¹C(=O)OR²- vs. -R¹OC(=O)R²-).

The polymers and oligomers of the invention possess anti-cancer (e.g., anti-neoplastic) activity and can be used to treat cancer in an animal. The polymers and oligomers can be used in methods of treating cancer in an animal, in methods of reducing cancer in an animal, in methods of treating or preventing the spread or metastasis of cancer in an animal, or in methods of treating an animal afflicted with a tumor or with cancer.

The term "treatment of cancer" or "treating cancer" refers to the prevention or alleviation or amelioration of any of the specific phenomena known in the art to be associated with the pathology commonly known as "cancer." The term "cancer" refers to the spectrum of pathological symptoms associated with the initiation or progression, as well as metastasis, of malignant tumors. By the term "tumor" is intended, for the purpose of the present invention, a new growth of tissue in which the multiplication of cells is uncontrolled and progressive. The tumor that is particularly relevant to the invention is the malignant tumor, one in which the primary tumor has the properties of invasion or metastasis or which shows a greater degree of anaplasia than do benign tumors. Thus, "treatment of cancer" or "treating cancer" refers to an activity that prevents, alleviates or ameliorates any of the primary phenomena (initiation, progression, metastasis) or secondary symptoms associated with the disease.

Cancers that are treatable are broadly divided into the categories of carcinoma, lymphoma and sarcoma. Examples of carcinomas that can be treated by the polymers and oligomers of the present invention include, but are not limited to: adenocarcinoma, acinic cell adenocarcinoma, adrenal cortical carcinomas, alveoli cell carcinoma, anaplastic carcinoma, basaloid carcinoma, basal cell carcinoma, bronchiolar carcinoma, bronchogenic carcinoma, renaladinol carcinoma, embryonal carcinoma, anometroid carcinoma, fibrolamolar liver cell carcinoma, follicular carcinomas, giant cell carcinomas, hepatocellular carcinoma, intraepidermal carcinoma, intraepithelial carcinoma, leptomanigio carcinoma, medullary carcinoma, melanotic carcinoma, menigual carcinoma, mesometonephric carcinoma, oat cell carcinoma, squamal cell carcinoma, sweat gland carcinoma, transitional cell carcinoma, and tubular cell carcinoma. Sarcomas that can be treated by the polymers and oligomers of the present invention include, but are not limited to: amelioblastic sarcoma, angiolithic sarcoma, botryoid sarcoma, endometrial stroma sarcoma, ewing sarcoma, fascicular sarcoma, giant cell sarcoma, granulositic sarcoma, immunoblastic sarcoma, juxaccordial osteogenic sarcoma, coppices sarcoma, leukocytic sarcoma (leukemia), lymphatic sarcoma (lympho sarcoma), medullary sarcoma, myeloid sarcoma (granulocitic sarcoma), austiogenci sarcoma, periosteal sarcoma, reticulum cell sarcoma (histiocytic lymphoma), round cell sarcoma, spindle cell sarcoma, synovial sarcoma, and telangiectatic audiogenic sarcoma. Lymphomas that can be treated by the polymers and oligomers of the present invention include, but are not limited to: Hodgkin's disease and lymphocytic lymphomas, such as Burkitt's lymphoma, NPDL, NML, NH and diffuse lymphomas.

Thus, examples of cancers that can be treated using the polymers and oligomers of the present invention include, but are not limited to, Hodgkin's disease, non-Hodgkin's lymphomas, acute lymphocytic leukemia, multiple myeloma, breast carcinomas, ovarian carcinomas, lung carcinomas, Wilms' tumor, testicular carcinomas, soft-tissue sarcomas, chronic lymphocytic leukemia, primary macroglobulinemia, bladder carcinomas, chronic granulocytic leukemia, primary brain carcinomas, malignant melanoma, small-cell lung carcinomas, stomach carcinomas, colon carcinomas, malignant pancreatic insulinoma, malignant carcinoid carcinomas, malignant melanomas, choriocarcinomas, mycosis fungoides, head and neck carcinomas, osteogenic sarcoma, pancreatic carcinomas, acute granulocytic leukemia, hairy cell leukemia, rhabdomyosarcoma, Kaposi's sarcoma, genitourinary carcinomas, thyroid carcinomas, esophageal carcinomas, malignant hypercalcemia, renal cell carcinomas, endometrial carcinomas, polycythemia vera, essential thrombocytosis, adrenal cortex carcinomas, skin cancer, and prostatic carcinomas.

Thus, in some aspects, the present invention is directed to a method of treating cancer in an animal in need thereof, the method comprising administering to the animal an effective amount of a pharmaceutical composition comprising a polymer of oligomer described above, for example, a polymer or oligomer of Formula I, Formula II, Formula IIa, Formula IV, Formula IVa, Formula IVb, Formula IVc, Formula V, or Formula Va, as defined above, and a pharmaceutically acceptable carrier or diluent.

In some aspects, the present invention is also directed to a method of reducing cancer in an animal, the method comprising administering to the animal an effective amount of a polymer or an oligomer described above, for example, a polymer or oligomer of Formula I, Formula II, Formula IIa, Formula IV, Formula IVa, Formula IVb, Formula IVc, Formula V, or Formula Va, as defined above.

In other aspects, the present invention is directed to a method of treating an animal afflicted with a tumor or cancer, the method comprising administering to the animal an effective amount of a polymer or an oligomer described above, for example, a polymer or oligomer of Formula I, Formula II, Formula IIa, Formula IV, Formula IVa, Formula IVb, Formula IVc, Formula V, or Formula Va, as defined above.

The polymers and oligomers of the present invention appear to be useful in treating metastases.

Thus, in some aspects, the present invention is directed to a method of treating or preventing the spread or metastasis of cancer in an animal, the method comprising administering to the animal an effective amount of a polymer or an oligomer described above, for example, a polymer or oligomer of Formula I, Formula II, Formula IIa, Formula IV, Formula IVa, Formula IVb, Formula IVc, Formula V, or Formula Va, as defined above.

The polymers and oligomers of the present invention can also be used in methods of killing or inhibiting the growth of cancer cells, either *in vivo* or *in vitro,* or inhibiting the growth of a cancerous tumor.

Thus, in some aspects, the invention is also directed to a method of killing or inhibiting the growth of a cancer cell, the method comprising contacting the cancer cell with an effective amount of a polymer or oligomer described above, for example, a polymer or oligomer of Formula I, Formula II, Formula IIa, Formula IV, Formula IVa, Formula IVb, Formula IVc, Formula V, or Formula Va, as defined above.

In other aspects, the invention is directed to a method of inhibiting tumor growth, the method comprising contacting the tumor with an effective amount of a polymer or an oligomer described above, for example, a polymer or oligomer of Formula I, Formula II, Formula IIa, Formula IV, Formula IVa, Formula IVb, Formula IVc, Formula V, or Formula Va, as defined above.

The polymer or oligomer in any one of the above methods can be administered to a human subject. Thus, in some aspects of the invention, the polymer or oligomer is administered to a human.

The methods disclosed above also have veterinary applications and can be used to treat non-human vertebrates. Thus, in other aspects of the invention, the polymer or oligomer is administered in any one of the above methods to non-human vertebrates, such as wild, domestic, or farm animals, including, but not limited to, cattle, sheep, goats, pigs, dogs, cats, and poultry such as chicken, turkeys, quail, pigeons, ornamental birds and the like.

The preparation of the polymers and oligomers of the present invention are described in detail inWIPO Publication No. WO 2004/082634, and in U.S. Patent Appl. No. 11/038,787, the contents of each of which are fully incorporated by reference herein in its entirety.

Briefly, polyamide and polyester polymers and oligomers of the present invention can be prepared by typical condensation polymerization and addition polymerization processes. See, for example, G. Odian, Principles of Polymerization, John Wiley & Sons, Third Edition (1991), M. Steven, Polymer Chemistry, Oxford University Press (1999). Most commonly the polyamides are prepared by (a) thermal dehydration of amine salts of carboxylic acids, (b) reaction of acid chlorides with amines and (c) aminolysis of esters. The most common method for the preparation of polyureas is the reaction of diamines with diisocyanates. (Yamaguchi, I. et al., Polym. Bull. 44:247 (2000)) This exothermic reaction can be carried out by solution techniques or by interfacial techniques. One skilled in organic and polymer chemistry will appreciate that the diisocyanate can be replaced with a variety of other bis-acylating agents *e.g.*, phosgene or N, N'-(diimidazolyl)carbonyl, with similar results. Polyurethanes are prepared by comparable techniques using a diisocyanate and a dialcohol or by reaction of a diamine with a *bis-*chloroformate.

The polyaryl and polyarylalkynyl polymers and oligomers of the present invention are synthesized according to the procedures outlined in WIPO Publ. No. WO 02/072007 and U.S. Patent Application No. 11/038,787. The entire contents of both WIPO Publ. No. WO 02/072007 and U.S. Patent Application No. 11/038,787 are fully incorporated herein by reference.

The syntheses of appropriately substituted monomers in the polymers and oligomers of the invention are straightforward. Numerous pathways are available to incorporate polar and nonpolar side chains. For example, phenolic groups on the monomer can be alkylated. Alkylation of the commercially available phenol will be accomplished with standard Williamson ether synthesis for the non-polar side chain with ethyl bromide as the alkylating agent. Polar sidechains can be introduced with bifunctional alkylating agents such as BOC-NH(CH₂)₂Br. Alternatively, the phenol group can be alkylated to install the desired polar side chain function by employing the Mitsonobu reaction with BOC-NH(CH₂)₂-OH, triphenyl phosphine, and diethyl acetylenedicarboxylate. Standard conditions for reduction of the nitro groups and hydrolysis of the ester afford the amino acid. With the aniline and benzoic acid in hand, coupling can be effected under a variety of conditions. Alternatively, the hydroxy group of the (di)nitrophenol can be converted to a leaving group and a functionality introduced under nucleophilic aromatic substitution conditions. Other potential scaffolds that can be prepared with similar sequences are methyl 2-nitro-4-hydroxybenzoate and methyl 2-hydroxy-4-nitrobenzoate.

The polymers and oligomers of the present invention are designed using computer-aided computational techniques, such as *de novo* design techniques, to embody the amphiphilic properties believed to be important for activity. In general, *de novo* design of oligomers is done by defining a three-dimensional framework of the backbone assembled from a repeating sequence of monomers using molecular dynamics and quantum force field calculations. Next, side groups are computationally grafted onto the backbone to maximize diversity and maintain drug-like properties. The best combinations of functional groups are then computationally selected to produce a cationic, amphiphilic structures. Representative compounds are synthesized from this selected library to verify structures and test their biological activity. Importantly, novel molecular dynamic and coarse grain modeling programs have been developed for this approach because existing force fields developed for biological molecules, such as peptides, were unreliable in these oligomer applications (Car, R., and Parrinello, M., Phys. Rev. Lett., 55:2471-2474 (1985); Siepmann, J.I., and Frenkel, D., Mol. Phys. 75:59-70 (1992); Martin, M.G., and Siepmann, J.I., J. Phys. Chem. B 103:4508-4517 (1999); Brooks, B.R., et al., J. Comp. Chem. 4:187-217 (1983)). Several chemical structural series of oligomers and polymers have been prepared. See, for example, WO 02/100295 A2, the entire contents of which are incorporated herein by reference. The polymers and oligomers of the present invention are prepared in a similar manner (see below).

The general approach is as follows:
1) A polymer backbone that should fold into a given, well-defined three-dimensional structure is defined. Extensive theoretical studies are carried out to demonstrate that the polymers are able to adopt the desired secondary conformation. Model compounds (short oligomers) are prepared for structural analysis of folding by X-ray crystallography.
2) The backbone of the polymer is then decorated with appropriate functional groups to endow the oligomer or polymer with the desired facial amphiphilic character.
3) The desired oligomers and polymers are synthesized, and their biological activities are measured.
4) Biophysical studies are carried out to confirm that the polymers are binding to membranes in the desired conformation and that the mechanism of action is as expected from the design.
5) Based on the findings, structures are redesigned to optimize the potency and selectivity of the compounds, and steps 2-4 are re-iterated.

The goal of this approach is to capture the structural and biological properties of antimicrobial peptides within the framework of traditional polymers that can be prepared by inexpensive condensation reactions.

An example of the design, synthesis, and testing of arylamide polymers and oligomers, a subgroup of polymers and oligomers disclosed in the present invention, is presented in Tew, G.N., et al., Proc. Natl. Acad. Sci. USA 99:5110-5114 (2002), the contents of which are fully incorporated by reference herein (Tew, G.N., et al., Proc. Natl. Acad. Sci. USA 99:5110-5114 (2002)). See also WIPO Publication No. WO 2004/082634, the contents of which are fully incorporated by reference herein in its entirety. Examples of the design, synthesis, and testing of phenylakynyl polymers and oligomers is presented in U.S. Patent Appl. No. 11/038,787, the contents of which are fully incorporated by reference herein in its entirety.

Oligomers of the present invention can be synthesized by solid-phase synthetic procedures well know to those of skill in the art. See, for example, Tew *et al.* (Tew, G.N., et al., Proc. Natl. Acad. Sci. USA 99:5110-5114 (2002)). See also Barany, G., et al., Int. J. Pept. Prot. Res. 30:705-739 (1987); Solid-phase Synthesis: A Practical Guide, Kates, S.A., and Albericio, F., eds., Marcel Dekker, New York (2000); and Dörwald, F.Z., Organic Synthesis on Solid Phase: Supports, Linkers, Reactions, 2nd Ed., Wiley-VCH, Weinheim (2002).

The polymers and oligomers of the invention are synthesized in a range of molecular weights. Molecular weights for the polymers and oligomers range from about 300 Daltons to about 1,000,000 Daltons. Preferred polymers of the present invention have average molecular weights that range from about 400 Daltons to about 120,000 Daltons (about 2 to about 500 monomer units). Especially preferred polymers have average weights that range from about 1,000 Daltons to about 25,000 Daltons (about 5 to about 100 monomer units). Oligomers of the present invention have molecular weights that range from about 300 Daltons to about 6,000 Daltons (about 2 to about 25 monomer units), with preferred oligomers having molecular weights that range from about 300 Daltons to about 2,500 Daltons (about 2 to about 10 monomer units).

One of skill in the art will recognize that the synthetic processes for producing polymers and oligomers of the invention can be modified to produce different ranges in molecular weight. The polymer chemist will readily appreciate that the chain length of polymers can be varied by techniques know in the polymer art. Advancements in solid-phase and solution phase synthesis of amino acid oligomers have made available techniques to prepare homogeneous oligomers with defined sequence and size and these techniques can be adapted to the present invention.

The polymers and oligomers of the invention are tested for anti-cancer activity by methods known to those of skill in the art. Examples of anti-cancer assays include, but are not limited to, standard cell viability assays, such as the XTT assay described in Example 1 below, or by metabolic activity assays.

The polymers and oligomers of the present invention are administered in the conventional manner by any route where they are active. Administration can be systemic, topical, or oral. For example, administration can be, but is not limited to, parenteral, subcutaneous, intravenous, intramuscular, intraperitoneal, transdermal, oral, buccal, or ocular routes, or intravaginally, by inhalation, by depot injections, or by implants. Thus, modes of administration for the polymers and oligomers (either alone or in combination with other pharmaceuticals) can be, but are not limited to, sublingual, injectable (including short-acting, depot, implant and pellet forms injected subcutaneously or intramuscularly), or by use of vaginal creams, suppositories, pessaries, vaginal rings, rectal suppositories, intrauterine devices, and transdermal forms such as patches and creams.

Specific modes of administration will depend on the indication (*e.g.*, whether the polymer or oligomer is administered to treat a microbial infection, or to provide an antidote for hemorrhagic conditions associated with heparin therapy). The mode of administration can depend on the pathogen or microbe to be targeted. The selection of the specific route of administration and the dose regimen is to be adjusted or titrated by the clinician according to methods known to the clinician in order to obtain the optimal clinical response. The amount of polymer or oligomer to be administered is that amount which is therapeutically effective. The dosage to be administered will depend on the characteristics of the subject being treated, *e.g.*, the particular animal treated, age, weight, health, types of concurrent treatment, if any, and frequency of treatments, and can be easily determined by one of skill in the art (*e.g.*, by the clinician).

The pharmaceutical formulations containing the polymers and oligomers and a suitable carrier can be solid dosage forms which include, but are not limited to, tablets, capsules, cachets, pellets, pills, powders and granules; topical dosage forms which include, but are not limited to, solutions, powders, fluid emulsions, fluid suspensions, semi-solids, ointments, pastes, creams, gels and jellies, and foams; and parenteral dosage forms which include, but are not limited to, solutions, suspensions, emulsions, and dry powder; comprising an effective amount of a polymer or oligomer as taught in this invention. It is also known in the art that the active ingredients can be contained in such formulations with pharmaceutically acceptable diluents, fillers, disintegrants, binders, lubricants, surfactants, hydrophobic vehicles, water soluble vehicles, emulsifiers, buffers, humectants, moisturizers, solubilizers, preservatives and the like. The means and methods for administration are known in the art and an artisan can refer to various pharmacologic references for guidance. For example, Modern Pharmaceutics, Banker & Rhodes, Marcel Dekker, Inc. (1979); and Goodman & Gilman's The Pharmaceutical Basis of Therapeutics, 6th Edition, MacMillan Publishing Co., New York (1980) can be consulted.

The polymers and oligomers can be formulated for parenteral administration by injection, *e.g.*, by bolus injection or continuous infusion. The polymers and oligomers can be administered by continuous infusion subcutaneously over a period of about 15 minutes to about 24 hours. Formulations for injection can be presented in unit dosage form, *e.g.*, in ampoules or in multi-dose containers, with an added preservative. The compositions can take such forms as suspensions, solutions or emulsions in oily or aqueous vehicles, and can contain formulatory agents such as suspending, stabilizing and/or dispersing agents.

For oral administration, the polymers and oligomers can be formulated readily by combining these compounds with pharmaceutically acceptable carriers well known in the art. Such carriers enable the compounds of the invention to be formulated as tablets, pills, dragees, capsules, liquids, gels, syrups, slurries, suspensions and the like, for oral ingestion by a patient to be treated. Pharmaceutical preparations for oral use can be obtained by adding a solid excipient, optionally grinding the resulting mixture, and processing the mixture of granules, after adding suitable auxiliaries, if desired, to obtain tablets or dragee cores. Suitable excipients include, but are not limited to, fillers such as sugars, including, but not limited to, lactose, sucrose, mannitol, and sorbitol; cellulose preparations such as, but not limited to, maize starch, wheat starch, rice starch, potato starch, gelatin, gum tragacanth, methyl cellulose, hydroxypropylmethyl-cellulose, sodium carboxymethylcellulose, and polyvinylpyrrolidone (PVP). If desired, disintegrating agents can be added, such as, but not limited to, the cross-linked polyvinyl pyrrolidone, agar, or alginic acid or a salt thereof such as sodium alginate.

Dragee cores can be provided with suitable coatings. For this purpose, concentrated sugar solutions can be used, which can optionally contain gum arabic, talc, polyvinyl pyrrolidone, carbopol gel, polyethylene glycol, and/or titanium dioxide, lacquer solutions, and suitable organic solvents or solvent mixtures. Dyestuffs or pigments can be added to the tablets or dragee coatings for identification or to characterize different combinations of active compound doses.

Pharmaceutical preparations which can be used orally include, but are not limited to, push-fit capsules made of gelatin, as well as soft, sealed capsules made of gelatin and a plasticizer, such as glycerol or sorbitol. The push-fit capsules can contain the active ingredients in admixture with filler such as, *e.g.*, lactose, binders such as, *e.g.*, starches, and/or lubricants such as, *e.g.*, talc or magnesium stearate and, optionally, stabilizers. In soft capsules, the active compounds can be dissolved or suspended in suitable liquids, such as fatty oils, liquid paraffin, or liquid polyethylene glycols. In addition, stabilizers can be added. All formulations for oral administration should be in dosages suitable for such administration.

For buccal administration, the polymer and oligomer compositions can take the form of, *e.g.*, tablets or lozenges formulated in a conventional manner.

For administration by inhalation, the polymers and oligomers for use according to the present invention are conveniently delivered in the form of an aerosol spray presentation from pressurized packs or a nebulizer, with the use of a suitable propellant, *e.g.*, dichlorodifluoromethane, trichlorofluoromethane, dichlorotetrafluoroethane, carbon dioxide or other suitable gas. In the case of a pressurized aerosol the dosage unit can be determined by providing a valve to deliver a metered amount. Capsules and cartridges of, *e.g.*, gelatin for use in an inhaler or insufflator can be formulated containing a powder mix of the compound and a suitable powder base such as lactose or starch.

The polymers and oligomers can also be formulated in rectal compositions such as suppositories or retention enemas, *e.g.*, containing conventional suppository bases such as cocoa butter or other glycerides.

In addition to the formulations described previously, the polymers and oligomers can also be formulated as a depot preparation. Such long acting formulations can be administered by implantation (for example subcutaneously or intramuscularly) or by intramuscular injection.

Depot injections can be administered at about I to about 6 months or longer intervals. Thus, for example, the compounds can be formulated with suitable polymeric or hydrophobic materials (for example as an emulsion in an acceptable oil) or ion exchange resins, or as sparingly soluble derivatives, for example, as a sparingly soluble salt.

In transdermal administration, the polymers and oligomers, for example, can be applied to a plaster, or can be applied by transdermal, therapeutic systems that are consequently supplied to the organism.

The pharmaceutical compositions of the polymers and oligomers also can comprise suitable solid or gel phase carriers or excipients. Examples of such carriers or excipients include but are not limited to calcium carbonate, calcium phosphate, various sugars, starches, cellulose derivatives, gelatin, and polymers such as, *e.g.*, polyethylene glycols.

The polymers and oligomers can also be administered in combination with other active ingredients, such as, for example, other anti-cancer or anti-neoplastic agents, or in combination with other cancer therapies other than chemotherapy, such as, for example, surgery or radiotherapy.

The following examples will serve to further typify the nature of this invention but should not be construed as a limitation in the scope thereof, which scope is defined solely by the appended claims.

### EXAMPLE 1

Compound 1 (shown below) was tested for its effectiveness against breast cancer cells. Compound 1 has an extended backbone structure with the cationic charges separated from the hydrophobic backbone by two methylene units. This compound has demonstrated potent cytotoxicity against a broad spectrum of bacteria, including *E. coli* D31, *B. anthracis* ATTC 1099, and *S*. *typhymurium* ATTC 29631, within a range of 0.8 to 1.6 µg/mL. Compound 1 also shows significant selectivity towards bacteria: When Compound 1 is tested against human red blood cells, 50% lysis (HC₅₀) occurs at a concentration of 75 µg/ml.

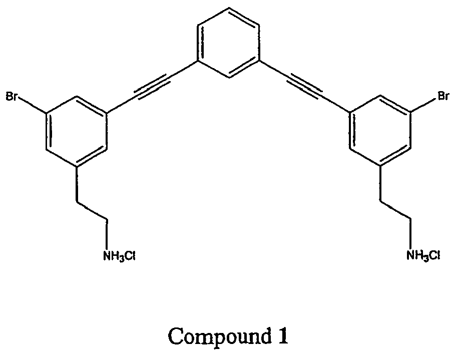

Compound 1 was tested against two human breast cancer cell lines, MCF-7 (ATCC HTB-22) and TMX2-28, and one non-tumorigenic breast cell line, MCF-10A (ATCC CRL-10317). MCF-7 and TMX2-28 cells were grown in DC₅ cell growth media while the MCF-10A cells were grown in MEGM, both supplemented with 5% bovine growth serum. The cells were grown using standard techniques. Cell culures at 50% confluence were harvested with trypsin, seeded onto sterile 96 well plates at a density of 10,000 cells/well and allowed to grow overnight to 50% confluence. Compound 1 was then added to the growth medium and allowed to further incubate for 48 hours. Viable cells were quantitated using an XTT assay (purchased from Roche).

The results of the study are presented in Table 1.

**Table 1:**

| Growth inhibition of cancerous and normal cell types by Compound 1. | | | |
|---|---|---|---|
| **Cell Line** | **Cell Type** | **IC90** | **Selectivity IC₉₀/IC₉₀MCF10A** |
| MCF-7 | tumorigenic | 6.3 | 2 |
| TMX2-28 | tumorigenic | 6.3 | 2 |
| MCF10A | non-tumorigenic | 12.5 | - |

Cytotoxic activity of Compound 1 against tumorigenic and non-tumorigenic breast cell lines is shown in Table 1. When tested against the tumorigenic cell lines, MCF-7 and TMX2-28, Compound 1 maximally inhibits cell growth (IC₉₀) at concentrations of 6.3 µg/ml. Against the non-tumorigenic MCF-10A cells, the IC₉₀ is 12.5 µg/mL. This shows that there is a 2-fold selectivity towards cancerous cells over normal cells.

The results of the study demonstrate that compounds of the present invention are selectively cytotoxic for tumor cells over normal cells.

Having now fully described this invention, it will be understood to those of ordinary skill in the art that the same can be performed within a wide and equivalent range of conditions, formulations, and other parameters without affecting the scope of the invention or any embodiment thereof. All documents, e.g., scientific publications, patents, patent applications and patent publications recited herein are hereby incorporated by reference in their entirety to the same extent as if each individual document was specifically and individually indicated to be incorporated by reference in its entirety. Where the document cited only provides the first page of the document, the entire document is intended, including the remaining pages of the document.

## Claims

1. Use of an oligomer of Formula Va,
**R¹-A₁-s-A₂-s-A₁-R²** (Va)
or an acceptable salt or solvate thereof,
wherein:
A₁ and A₂ are independently optionally substituted arylene or optionally substituted heteroarylene, wherein:
(i) A₁ and A₂ are independently optionally substituted with one or more polar (PL) group(s), one or more non-polar (NPL) group(s), or a combination of one or more polar (PL) group(s) and one or more non-polar (NPL) group(s); or
(ii) one of A₁ or A₂ is as defined above and is optionally substituted with one or more polar (PL) group(s), one or more non-polar (NPL) group(s), or a combination of one or more polar (PL) group(s) and one or more non-polar (NPL) group(s); and the other of A₁ or A₂ is the group -C≡C(CH₂)ₚC≡C-, wherein p is 0 to 8, and the -(CH₂)ₚ- alkylene chain is optionally substituted with one or more amino or hydroxyl groups;
s is absent, or is -CH=CH- or -C ≡ C-;
R¹ is hydrogen, a polar group (PL), a non-polar group (NPL), or -s-A', wherein A' is aryl or heteroaryl, either of which is optionally substituted with one or more polar (PL) group(s), one or more non-polar (NPL) group(s), or a combination of one or more polar (PL) group(s) and one or more non-polar (NPL) group(s);
R² is R¹;
NPL is a nonpolar group independently selected from -B(OR⁴)₂ or -(NR^{3'})_{q1NPL}-U^{NPL}-(CH2)_{pNPL}-(NR^{3"})_{q2NPL}-R⁴, wherein
R³, R^{3'}, and R^{3"} are independently selected from the group consisting of hydrogen, alkyl, and alkoxy;
R⁴ is selected from the group consisting of hydrogen, alkyl, alkenyl, alkynyl, cycloalkyl, aryl, and heteroaryl, any of which is optionally substituted with one or more alkyl or halo groups;
U^{NPL} is absent or selected from the group consisting of O, S, S(=O), S(=O)₂, NR³, -(C=O)-, -(C=O)-N=N-NR³-, -(C=O)-NR³-N=N-, -N=N-NR³-, -C(=N-N(R³)₂)-, -C(=NR³)-, -C(=O)O-, -C(=O)S-, -C(=S)-, -O-P(=O)₂O-, -R³-O-, -R³-S-, -S-C=N- and -(C=O)-NR³-O-, wherein groups with two chemically nonequivalent termini can adopt both possible orientations;
the alkylene chain -(CH₂)_{pNPL}- is optionally substituted with one or more amino or hydroxyl groups, or the alkylene chain is unsaturated;
pNPL is 0 to 8;
q1NPL and q2NPL are independently 0 to 2;
PL is a polar group selected from the group consisting of halo, hydroxyethoxymethyl, methoxyethoxymethyl, polyoxyethylene, and -(NR^{5'})_{q1PL}-U^{PL}-(CH₂)_{pPL}-(NR^{5"})_{q2PL}-V, wherein;
R⁵, R^{5'}, and R^{5"} are independently selected from the group consisting of hydrogen, alkyl, and alkoxy;
U^{PL} is absent or selected from the group consisting of O, S, S(=O), S(=O)₂, NR⁵, -(C=O)-, -(C=O)-N=N-NR⁵-, -(C=O)-NR⁵-N=N-, -N=N-NR⁵-, -C(=N-N(R⁵)₂)-, -C(=NR⁵)-, -C(=O)O-, -C(=O)S-, -C(=S)-, -O-P(=O)₂O-, -R⁵O-, -R⁵S-, -S-C=N- and -(C=O)-NR⁵-O-, wherein groups with two chemically nonequivalent termini can adopt both possible orientations;
V is selected from the group consisting of nitro, cyano, amino, hydroxyl, alkoxy, alkylthio, alkylamino, dialkylamino, -NH(CH₂)pNH₂, -N(CH₂CH₂NH₂)₂, diazamino, amidino, guanidino, guanyl, semicarbazone, aryl, heterocycle and heteroaryl, any of which is optionally substituted with one or more of amino, halo, cyano, nitro, hydroxyl, -NH(CH₂)pNH₂, -N(CH₂CH₂NH₂)₂, amidino, guanidino, guanyl, aminosulfonyl, aminoalkoxy, aminoalkylthio, lower acylamino, or benzyloxycarbonyl;
the alkylene chain -(CH₂)_{pPL}- is optionally substituted with one or more amino or hydroxyl groups, or the alkylene chain is unsaturated;
pPL is 0 to 8; and
q1PL and q2PL are independently 0 to 2;
and a pharmaceutically acceptable carrier or diluent for the manufacture of a medicament for the treatment of cancer;
with the proviso that if A₁ and A₂ are thiophene, the polar groups cannot be 3-(propionic acid) or methoxy(diethoxy)ethyl and the nonpolar group cannot be n-dodecyl.

2. The use of claim 1, wherein said oligomer is one of 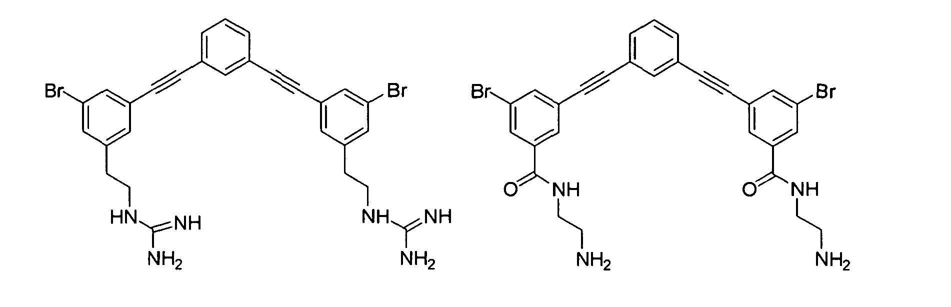 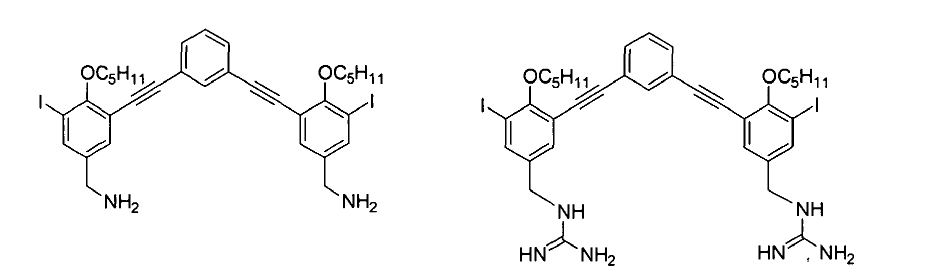 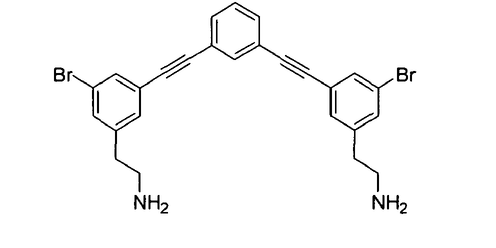 or physiologically acceptable salts thereof.

3. An in vitro method of killing or inhibiting the growth of a cancer cell, said method comprising contacting the cancer cell with an effective amount of the oligomer of Formula Va of claim 1.

4. Use of an oligomer of Formula V:
**R¹-[-A₁-s-A₂-s-]ₘ-R²** (V)
or an acceptable salt or solvate thereof,
wherein:
A₁ and A₂ are independently optionally substituted arylene or optionally substituted heteroarylene, wherein:
(i) A₁ and A₂ are independently optionally substituted with one or more polar (PL) group(s), one or more non-polar (NPL) group(s), or a combination of one or more polar (PL) group(s) and one or more non-polar (NPL) group(s); or (ii) one of A₁ or A₂ is as defined above and is optionally substituted with one or more polar (PL) group(s), one or more non-polar (NPL) group(s), or a combination of one or more polar (PL) group(s) and one or more non-polar (NPL) group(s); and the other of A₁ or A₂ is the group -C≡C(CH₂)ₚC=C-, wherein p is 0 to 8, and the -(CH₂)ₚ- alkylene chain is optionally substituted with one or more amino or hydroxyl groups;
s is absent, or represents ―CH₂-, ―CH₂-CH₂-, -CH=CH-, or -C≡C-;
R¹ is
(i) hydrogen, a polar group (PL), or a non-polar group (NPL), and R² is -A₁-R¹, wherein A₁ is as defined above and is optionally substituted with one or more polar (PL) group(s), one or more non-polar (NPL) group(s), or a combination of one or more polar (PL) group(s) and one or more non-polar (NPL) group(s); or
(ii) hydrogen, a polar group (PL), or a non-polar group (NPL), and R² is -A₁-s-A₂-R¹, wherein each of A₁, and A₂ is as defined above and is optionally substituted with one or more polar (PL) group(s), one or more non-polar (NPL) group(s), or a combination of one or more polar (PL) group(s) and one or more non-polar (NPL) group(s); or
(iii) A'-s- and R² is -A₁-s-A', wherein A' is aryl or heteroaryl, either of which is optionally substituted with one or more polar (PL) group(s), one or more non-polar (NPL) group(s), or a combination of one or more polar (PL) group(s) and one or more non-polar (NPL) group(s); or
(iv) A'-s- and R² is -A', wherein A' is aryl or heteroaryl, either of which is optionally substituted with one or more polar (PL) group(s), one or more non-polar (NPL) groups(s), or a combination of one or more polar (PL) group(s) and one or more non-polar (NPL) group(s); or
(iv) R¹ and R² together form a single bond;
NPL is a nonpolar group independently selected from -B(OR⁴)₂ or ―(NR^{3'})_{q1NPL}-U^{NPL}-(CH₂)_{pNPL}-(NR^{3"})_{q2NPL}-R⁴, wherein:
R³, R^{3'}, and R^{3"} are independently selected from the group consisting of hydrogen, alkyl, and alkoxy;
R⁴ is selected from the group consisting of hydrogen, alkyl, alkenyl, alkynyl, cycloalkyl, aryl, and heteroaryl, any of which is optionally substituted with one or more alkyl or halo groups;
U^{NPL} is absent or selected from the group consisting of O, S, S(=O), S(=O)₂, NR³, -(C=O)-, -(C=O)-N=N-NR³-, -(C=O)-NR³-N=N-, -N=N-NR³-, -C(=N-N(R³)₂)-, -C(=NR³)-, -C(=O)O-, -C(=O)S-, -C(=S)-, -O-P(=O)₂O-, -R³O-, -R³S-, -S-C=N- and -(C=O)-NR³-O-, wherein groups with two chemically nonequivalent termini can adopt both possible orientations;
the -(CH₂)_{pNPL}- alkylene chain is optionally substituted with one or more amino or hydroxyl groups, or the alkylene chain is unsaturated;
pNPL is 0 to 8;
q1NPL and q2NPL are independently 0 to 2;
PL is a polar group selected from the group consisting of halo, hydroxyethoxymethyl, methoxyethoxymethyl, polyoxyethylene, and -(NR^{5'})_{q1PL}-U^{PL}-(CH₂)_{pPL}-(NR^{5"})_{q2PL}-V, wherein:
R⁵, R^{5'}, and R^{5"} are independently selected from the group consisting of hydrogen, alkyl, and alkoxy;
U^{PL} is absent or selected from the group consisting of O, S, S(=O), S(=O)₂, NR⁵, -(C=O)-, -(C=O)-N=N-NR⁵-, -(C=O)-NR⁵-N=N-, -N=N-NR⁵-, -C(=N-N(R⁵)₂)-, -C(=NR⁵)-, -C(=O)O-, -C(=O)S-, -C(=S)-, -O-P(=O)₂O-, -R⁵O-, -R⁵S-, -S-C=N- and -(C=O)-NR⁵-O-, wherein groups with two chemically nonequivalent termini can adopt both possible orientations;
V is selected from the group consisting of nitro, cyano, amino, hydroxyl, alkoxy, alkylthio, alkylamino, dialkylamino, -NH(CH₂)ₚNH₂, -N(CH₂CH₂NH₂)₂, diazamino, amidino, guanidino, guanyl, semicarbazone, aryl, heterocycle and heteroaryl, any of which is optionally substituted with one or more of amino, halo, cyano, nitro, hydroxyl, -NH(CH₂)pNH₂, -N(CH₂CH₂NH₂)₂, amidino, guanidino, guanyl, aminosulfonyl, aminoalkoxy, aminoalkylthio, lower acylamino, or benzyloxycarbonyl;
the -(CH₂)_{pPL}- alkylene chain is optionally substituted with one or more amino or hydroxyl groups, or the alkylene chain is unsaturated;
pPL is 0 to 8;
q1PL and q2PL are independently 0 to 2; and
m is 1 to about 25; with the proviso that if A₁ and A₂ are thiophene, the polar groups cannot be 3-(propionic acid) or methoxy(diethoxy)ethyl and the nonpolar group cannot be n-dodecyl;
and a pharmaceutically acceptable carrier or diluent for the manufacture of a medicament for treating cancer.

5. Use of an oligomer of Formula V:
**R¹-[-A₁-s-A₂-s-]ₘ₋R²** (V)
or an acceptable salt or solvate thereof,
wherein:
A₁ and A₂ are independently optionally substituted arylene or optionally substituted heteroarylene, wherein:
(i) A₁ and A₂ are independently optionally substituted with one or more polar (PL) group(s), one or more non-polar (NPL) group(s), or a combination of one or more polar (PL) group(s) and one or more non-polar (NPL) group(s); or (ii) one of A₁ or A₂ is as defined above and is optionally substituted with one or more polar (PL) group(s), one or more non-polar (NPL) group(s), or a combination of one or more polar (PL) group(s) and one or more non-polar (NPL) group(s); and the other of A₁ or A₂ is the group -C≡C(CH₂)ₚC≡C-, wherein p is 0 to 8, and the -(CH₂)ₚ- alkylene chain is optionally substituted with one or more amino or hydroxyl groups;
s is absent, or represents ―CH₂-, ―CH₂-CH₂-, -CH=CH-, or -C ≡ C-;
R¹ is
(i) hydrogen, a polar group (PL), or a non-polar group (NPL), and R² is -A₁-R¹, wherein A₁ is as defined above and is optionally substituted with one or more polar (PL) group(s), one or more non-polar (NPL) group(s), or a combination of one or more polar (PL) group(s) and one or more non-polar (NPL) group(s); or
(ii) hydrogen, a polar group (PL), or a non-polar group (NPL), and R² is -A₁-s-A₂-R¹, wherein each of A₁, and A₂ is as defined above and is optionally substituted with one or more polar (PL) group(s), one or more non-polar (NPL) group(s), or a combination of one or more polar (PL) group(s) and one or more non-polar (NPL) group(s); or
(iii) A'-s- and R² is -A₁-s-A', wherein A' is aryl or heteroaryl, either of which is optionally substituted with one or more polar (PL) group(s), one or more non-polar (NPL) group(s), or a combination of one or more polar (PL) group(s) and one or more non-polar (NPL) group(s); or
(iv) A'-s- and R² is -A', wherein A' is aryl or heteroaryl, either of which is optionally substituted with one or more polar (PL) group(s), one or more non-polar (NPL) groups(s), or a combination of one or more polar (PL) group(s) and one or more non-polar (NPL) group(s); or
(v) R¹ and R² together form a single bond;
NPL is a nonpolar group independently selected from -B(OR⁴)₂ or -(NR^{3'})_{q1NPL}-U^{NPL}-(CH₂)_{pNPL}-(NR^{3"})_{q2NPL}-R⁴, wherein:
R³, R^{3'} , and R^{3"} are independently selected from the group consisting of hydrogen, alkyl, and alkoxy;
R⁴ is selected from the group consisting of hydrogen, alkyl, alkenyl, alkynyl, cycloalkyl, aryl, and heteroaryl, any of which is optionally substituted with one or more alkyl or halo groups;
U^{NPL} is absent or selected from the group consisting of O, S, S(=O), S(=O)₂, NR³, -(C=O)-, -(C=O)-N=N-NR³-, -(C=O)-NR³-N=N-, -N=N-NR³-, -C(=N-N(R³)₂)-, -C(=NR³)-, -C(=O)O-, -C(=O)S-, -C(=S)-, -O-P(=O)₂O-, -R³O-, -R³S-, -S-C=N- and -(C=O)-NR³-O-, wherein groups with two chemically nonequivalent termini can adopt both possible orientations;
the -(CH₂)_{pNPL}- alkylene chain is optionally substituted with one or more amino or hydroxyl groups, or the alkylene chain is unsaturated;
pNPL is 0 to 8;
q1NPL and q2NPL are independently 0 to 2;
PL is a polar group selected from the group consisting of halo, hydroxyethoxymethyl, methoxyethoxymethyl, polyoxyethylene, and -(NR^{5'})_{q1PL}-U^{PL}-(CH₂)_{pPL}-(NR^{5"})_{q2PL}-V, wherein:
R⁵, R^{5'} , and R^{5"} are independently selected from the group consisting of hydrogen, alkyl, and alkoxy;
U^{PL} is absent or selected from the group consisting of O, S, S(=O), S(=O)₂, NR⁵, -(C=O)-, -(C=O)-N=N-NR⁵-, -(C=O)-NR⁵-N=N-, -N=N-NR⁵-, -C(=N-N(R⁵)₂)-, -C(=NR⁵)-, -C(=O)O-, -C(=O)S-, -C(=S)-, -O-P(=O)₂O-, -R⁵O-, -R⁵S-, -S-C=N- and -(C=O)-NR⁵-O-, wherein groups with two chemically nonequivalent termini can adopt both possible orientations;
V is selected from the group consisting of nitro, cyano, amino, hydroxyl, alkoxy, alkylthio, alkylamino, dialkylamino, -NH(CH₂)pNH₂, -N(CH₂CH₂NH₂)₂, diazamino, amidino, guanidino, guanyl, semicarbazone, aryl, heterocycle and heteroaryl, any of which is optionally substituted with one or more of amino, halo, cyano, nitro, hydroxyl, -NH(CH₂)pNH₂, -N(CH₂CH₂NH₂)₂, amidino, guanidino, guanyl, aminosulfonyl, aminoalkoxy, aminoalkylthio, lower acylamino, or benzyloxycarbonyl;
the -(CH₂)_{pPL}- alkylene chain is optionally substituted with one or more amino or hydroxyl groups, or the alkylene chain is unsaturated;
pPL is 0 to 8;
q1PL and q2PL are independently 0 to 2; and
m is 1 to at least about 100;
with the proviso that if A₁ and A₂ are thiophene, the polar groups cannot be 3-(propionic acid) or methoxy(diethoxy)ethyl and the nonpolar group cannot be n-dodecyl;
for the manufacture of a medicament for killing or inhibiting the growth of a cancer cell.

6. Use of an oligomer of Formula Va or V according to claim 1 or claim 4 for the manufacture of a medicament for reducing cancer in an animal.

7. Use of an oligomer of Formula Va or V according to claim 1 or claim 4 for the manufacture of a medicament for inhibiting tumor growth.

8. Use of an oligomer of Formula Va or V according to claim 1 or claim 4 for the manufacture of a medicament for treating or preventing the spread or metastasis of cancer in an animal.

9. Use an oligomer of Formula Va or V according to claim 1 or claim 4 for the manufacture of a medicament for treating an animal afflicted with a tumor or cancer.
